# EUROPEAN PATENT SPECIFICATION

(11) **EP 3 201 900 B1**
(45) Date of publication and mention of the grant of the patent: **30.11.2022**
(21) Application number: 15847330.6
(22) Date of filing: 05.10.2015
(51) Int. Cl.: G09B 23/28

(54) **MEDICAMENT TRAINING DEVICE AND SYSTEM**
ARZNEIMITTELÜBUNGSVORRICHTUNG UND SYSTEM
DISPOSITIF ET SYSTÈME D'APPRENTISSAGE DE MÉDICAMENT

(30) Priority: 03.10.2014 US 201414505935
(43) Date of publication of application: 09.08.2017
(73) Proprietor: Baker, Jeff, Orlando, FL 32839 (US); Van der Pol, Paul, Winter Garden, FL 34787 (US); Siemer, Francis Michael, Orlando, FL 32828 (US); Baker, Chris, Lebanon, OH 45036 (US); Bunker, Mark, Orlando, FL 32801 (US); Marulanda, Karina, Orlando, FL 32836 (US); Palyo, Matthew, Orlando, FL 32801 (US); Freytag, Seth, Winter Springs, FL 32708 (US); Shuang, Hou Shi, Ningbo 315012 (CN); Chung, Christopher, Orlando, FL 32827 (US)
(72) Inventor: Baker, Jeff, Orlando, FL 32839 (US); Van der Pol, Paul, Winter Garden, FL 34787 (US); Siemer, Francis Michael, Orlando, FL 32828 (US); Baker, Chris, Lebanon, OH 45036 (US); Bunker, Mark, Orlando, FL 32801 (US); Marulanda, Karina, Orlando, FL 32836 (US); Palyo, Matthew, Orlando, FL 32801 (US); Freytag, Seth, Winter Springs, FL 32708 (US); Shuang, Hou Shi, Ningbo 315012 (CN); Chung, Christopher, Orlando, FL 32827 (US)
(74) Representative: ABG Intellectual Property Law, S.L.
(86) International application number: PCT/US2015/053992
(87) International publication number: WO 2016/054634

(56) References cited:
- WO-A2-2014/145535
- US-A1- 2013 266 919
- US-B2- 6 877 658
- US-B2- 8 622 973
- BAKER: 'Education patients on self-administered drug injections' January 2014, XP055424665 Retrieved from the Internet: <URL:http://www.pharmaceuticalcommerce.com/ index.php?pg=manufacturing_and_packagi ng&articleid=27079&keyword=self-administere d-drug-injections-patient-educat non%20resources>

## Description

### BACKGROUND

Performing a medical treatment or test on oneself carries with it certain risks and often creates a level of anxiety for the user performing the treatment or test. It has proven beneficial in the medical field to practice various medical techniques including drug delivery, specifically where it relates to injections and other invasive drug delivery means prior to delivering the medications to a patient in need, and particularly in the case of self-administration of medicaments. Training devices are helpful in reducing errors and anxiety associated with self administering medical treatment, as well as increasing efficiency and accuracy in providing the treatment to patients. Medical devices can be intimidating to use; the fear associated with giving oneself an injection, for example, can be traumatic. This fear is increased in persons with little or no experience in self-administration of medications. Consequently, devices and methods to assist in training individuals to inject themselves or otherwise self-administer medication are beneficial in decreasing or preventing the anxiety associated with medicament delivery. Medicament delivery training devices allow patients to practice giving themselves a full dose in a safe and effective manner.

Document US2013/266919A1 discloses a medicament delivery training device.

Document WO2014/145535A2 discloses a medicament delivery and simulation system with a removable disposable container for medicament delivery and training.

### SUMMARY

According to the invention, a medicament system is provided as defined in claim 1. The dependent claims define embodiments of the medicament system.

The claimed medicament system is configured to receive and/or communicate information about a medicament device or about a use of the medicament device, or a combination thereof, to a user, said medicament system comprising:
a medicament device comprising a housing and a mechanism comprising one or more mechanical components that move relative to one another to produce a mechanical sound during use;
a collateral device to provide information to a user, said collateral device comprising an information detecting and/or receiving component and a collateral device housing comprising an opening for receiving the medicament device, wherein the information detecting and/or receiving component comprises a microphone configured to receive an audible signal from the medicament device and/or the mechanical sound produced by the mechanism, the collateral device further comprising:
   a signal output component;
   a microprocessor;
   a power source; and
   a sensor that detects contact of the collateral device against the skin of a user;
   wherein said collateral device is configured to detect an error condition of the medicament device in response to information received by the microphone or information received by the microphone and information sensed by the sensor, wherein the error condition is incorrect positioning between the device and the user, incorrect contact between the device and the user, an out of order step and/or a step that exceeds or fails to meet a predetermined time value; and
   wherein the signal output component provides an output comprising information about the medicament system and/or a usage of the medicament device and/or medicament system to a user of the system.

It is also provided a method of using a collateral device to train a user of a medicament device to properly operate the medicament device to dispense a dose of medicament and to provide an instruction and/or a feedback to a user of the collateral device. The method may include detecting and/or receiving information from a medicament device, processing information received from the medicament device, and providing a signal output to a user based on information received and processed.

### BRIEF DESCRIPTION OF THE DRAWINGS

A more particular description briefly stated above will be rendered by reference to specific embodiments thereof that are illustrated in the appended drawings. Understanding that these drawings depict only typical embodiments and are not therefore to be considered to be limiting of its scope, the embodiments will be described and explained with additional specificity and detail through the use of the accompanying drawings in which:
FIG. 1 is a schematic of a system embodiment described herein.
FIG. 2 is a schematic of another system embodiment described herein.
FIG. 3 is a schematic of a further system embodiment described herein.
FIG. 4 is a schematic of a non-claimed system example described herein.
FIG. 5 is a schematic of a system embodiment described herein.
FIG. 6 is a schematic of a system embodiment described herein.
FIGS. 7A-7E include views of an embodiment of a system described herein, wherein a collateral device is shown in cross-sectional view.
FIG. 7F is an exploded view of the collateral device embodiment shown in FIGS. 7A-7E.
FIGS. 8A-B are views of another embodiment of a system described herein, wherein a collateral device is shown in cross-sectional view.
FIG. 8C is an exploded view of the collateral device embodiment shown in FIG. 8A-B.
FIG. 9A-E are various side, top, bottom, front and back views of an embodiment of a collateral device.
FIG. 9F is an exploded view of the collateral device embodiment shown in FIGS. 9 A-E.
FIG. 9G is a perspective view of the collateral device embodiment shown in FIGS. 9A-F attached onto a medicament device.
FIG. 10A is a perspective view of another embodiment of a collateral device associated with a medicament device.
FIG. 10B is an exploded view of the embodiment of the collateral device shown in FIG. 10A.
FIGS. 11A-C are side views of an embodiment of a system described herein, including a cross-sectional view of a further embodiment of a collateral device.

### DETAILED DESCRIPTION

For the purposes of promoting an understanding of the principles and operation of the invention, reference will now be made to the embodiments illustrated in the drawings and specific language will be used to describe the same.

It is important to an understanding of the present invention to note that all technical and scientific terms used herein, unless defined herein, are intended to have the same meaning as commonly understood by one of ordinary skill in the art. The techniques employed herein are also those that are known to one of ordinary skill in the art, unless stated otherwise. For purposes of more clearly facilitating an understanding the invention as disclosed and claimed herein, the following definitions are provided.

In addition to increasing confidence in self-administration in users by practicing with a medicament system, the inventors have identified additional benefits associated with multi-sensory learning regarding a medicament system. It has been discovered that multi-sensory learning establishes multiple pathways in separate areas in the brain and ultimately results in a highly effective learning experience. However, in order to gain benefits from multi-sensory learning devices, certain requirements must be met including but not limited to the following: the sources of stimuli must be in close proximity to one another; the sources of stimuli must be synchronous; the stimuli must be congruous semantically, otherwise the superior colliculus (area of the brain located in the midbrain known for integrating multiple sources of information) will segregate the stimuli instead of integrate them; and finally, the use of extraneous materials must be limited.

With knowledge of the essential factors in multi-sensory learning and incorporation of the multi-sensory learning features into a training system, the inventors have developed a novel, cutting-edge medicament system. The inventors have identified a need for a system to monitor users and their use of a medicament device, assist users in administering medication, or training for medication administration. Various modes of administration are provided by the system embodiments disclosed herein including parenterally administered medications, inhaler-based medications, among other modes of administration. Embodiments of the system described in greater detail below include applications for home use, in-office use by health care provider (HCP) or by the patient, hospital use, and educational use for training medical professionals and other personnel among other potential uses. The inventors have discovered a system for training individuals to use medical devices while improving user comfort and confidence in delivery and administration of medicament.

Exemplary embodiments of the medicament delivery training device can be implemented to educate users on the proper operation and usage of a medicament device. The medicament system can be used to make prospective and current users of medicament devices feel more comfortable and confident in self-administration (or administration to others) of medicaments, and can help users understand the proper steps of medicament delivery. Exemplary embodiments of the medicament system can be used by a user before the user administers a medication by way of, for example, an auto-injector by way of using an actual automatic injection device corresponding to the automatic injection training device and/or can be used as needed or desired by the user. Other exemplary embodiments of the invention herein pertain to manual injection devices and manual injection training devices used by the user, respiratory inhaler trainers and respiratory inhaler drug delivery devices, in non-limiting examples.

The medicament system takes advantage of the multisensory learning capabilities of the human brain. As such, the medicament system provides the means to stimulate primarily the visual, auditory and somatic systems of the human nervous system.

Visual stimuli or feedback (visual output) can be generated mechanically or electronically. An example of a mechanically generated visual stimulus is a plunger moving past an inspection window in an autoinjector or prefilled syringe medicament device or a shroud extending from an injection device. An example of an electronically generated visual stimulus is one or more LED's blinking, an LCD display showing an icon, or key steps in the process of administration being highlighted on a screen in the order required for proper administration of medicament, in non-limiting examples. A visual output as disclosed herein includes but is not limited to a light, a display, a colorometric display system, a change in position of the device or any other type of visual cue to the user of the container and/or device. The visual output is associated with the medicament device or with the medicament training container; therefore it may be disposed on either portion of the system or provided in connection with the system either by a wire or wirelessly.

Additional visual outputs that may be incorporated into the system herein may include display devices having one or more layers of material having a light transmission region, a unit of information to be highlighted, and a light blocking region; and a backlight unit having a flexible, planar waveguide body, a light source configured to direct light into the waveguide body, and at least one light director associated with a portion of the waveguide body so as to direct light transversely to a plane of the waveguide body. The directed light travels through the light transmission region, and the directed light is directed toward the unit of information to be highlighted as provided in International Application No. PCT/US11/26976 and US National Stage Application Serial No. 13/582,560 which claim the benefit of US Provisional Application Serial No. 61/310,081. The unit or units of information to be highlighted may include the stepwise instructions for administering the medicament to a user and may also provide the duration of each step by way of highlighting each step for a predetermined amount of time such that the user can follow the precise timing of each step in the sequence.

Auditory stimuli or feedback (audio output) can also be generated mechanically or electronically. An example of a mechanically generated auditory stimulus is the "click" that can be heard if two parts of a device interlock. An example of an electronically generated auditory stimulus is a beeper or a speaker that plays spoken instructions. An audio output as disclosed herein can be generated mechanically or electronically, for example, and includes but is not limited to music, a sound, a beep, a series of beeps music or sounds, a mechanical sound including clicking, the movement of one or more parts of a medicament device relative to one another, or a sound replication of operation or behavior of a drug delivery device containing medicament. These auditory stimuli, such as two parts of a device interlocking can be picked up by a microphone of the system. According to the claimed invention, the collateral device comprises an information detecting and/or receiving component, wherein the information detecting and/or receiving component comprises a microphone. The microphone may receive audio input from the medicament device as described above during use of the system. The system can then identify whether or not the device was used correctly (i.e., whether a step was performed correctly or in the correct order, for example). A combination of both visual and auditory output may include a video tutorial providing instructions to a user on proper administration of the medicament or use of the training device, for example.

Somatic stimuli or feedback, also called somatosensory stimuli or tactile feedback, is typically generated mechanically. In a typical embodiment of the medicament system, there are a large number of somatic stimuli, particularly with reference to the medicament device, such as actuation forces, abrasion resistance, frictional forces, spring compression, the feel of a click if two parts interlocking, surface texture, vibrations, weight sensation, and any other similar stimuli or feedback known to those of skill in the art.

A "predetermined value" as used herein, for example, includes but is not limited to a value or range of values relating to an event involving use or operation of the device. These may include, but are not limited to thresholds, ceilings, baselines or range values that are desired or undesired for a particular event. Examples of predetermined values include, but are not limited to, a predetermined orientation value, predetermined time value, or a predetermined contact value, in addition to other predetermined values described herein refers to a value that is used as a reference value in relation to a value, signal, or indication that is detected by, for example, a sensor of the medicament device. Predetermined value may include an optimal value, or a sub-optimal value, or any value there between, or any combination thereof. The term "value" as used herein, may refer to a specific value or a range of values.

In one example, a predetermined perpendicularity value may include a 90 degree angle between the device and a target region for the medicament device, an additional predetermined perpendicularity value may include a 10 degree angle between the device and a target region for the device. At either predetermined perpendicularity value, or at any value there between, a signal output component may be initiated. The signal output component may therefore be an error message or a congratulatory message, for example. This signal output component may be initiated from the medicament device and/or the medicament training container.

The term "condition" as used herein includes but is not limited to one or a combination of a user input, a status of the medicament device or the medicament training container, anything that is sensed by the device or container, correct or incorrect stepwise activities, usage of the device over time, among other conditions.

According to the claimed invention, the "error condition" is one or a combination of an incorrect positioning or contact between the device and the user, an out of order step, or a step that exceeds or fails to meet predetermined time value (such as an undue pause during or between steps, or insufficient time for conducting a step or transition between steps).

The term reconstituted as used herein includes a return of the components to their original state. For example, following use of the medicament device, once the device is in a post-delivery state (or post training state), it can be reset for subsequent use. As part of the resetting of the device from a post-delivery (or post-training) state to a pre-delivery (or pre-training) state, the signal output components including audio, visual, olfactory, gustatory, and tactile are also reset back to their original states, or reconstituted, such that a subsequent training or medicament delivery can be performed with the device. The term reconstituted may also include return of the medicament training container to its original state and may include a return of the stepwise instructions to the first step in the sequence or a replacement of the medicament device within the medicament training container in preparation for a subsequent medicament delivery or training, for example.

The term "fluid" as used herein may include any type of fluid, including but not limited to liquid or gas. The fluid may specifically include a liquid, powder or aerosol medicament, air flowing to or from the user, or liquid coming from the user, in some non-limiting embodiments.

The terminology used herein is for the purpose of describing particular embodiments only and is not intended to be limiting. Furthermore, to the extent that the terms "including," "includes," "having," "has," "with," or variants thereof are used in either the detailed description and/or the claims, such terms are intended to be inclusive in a manner similar to the term "comprising." Moreover, unless specifically stated, any use of the terms first, second, etc., does not denote any order, quantity or importance, but rather the terms first, second, etc., are used to distinguish one element from another.

The term "sensor" or "sensors" as used herein may include but are not limited to, light sensors, fluid flow rate sensors, strain gauge sensors, temperature sensors, pressure sensors, tilt sensors, force sensors, level sensors, contact sensors, photoelectric sensors, magnetic sensors, ultrasonic sensors, electrochemical sensors, acceleration sensors, moisture sensors, humidity sensors, speed sensors, inductive sensors, capacitive sensors, and orientation sensors. Some of these sensors may require a supply of voltage. The medicament system may include one or more of the sensors described herein. According to the claimed invention, the collateral device comprises a sensor that detects contact of the collateral device against the skin of a user. The collateral device may further comprise a sensor configured to detect when the medicament device distal end is received within the opening of the collateral device housing for receiving the medicament device.

In an example, two or more contact sensors, more preferably three contact sensors may be used to detect perpendicularity of the medicament device relative to a target area of a patient. An example of an inductive sensor includes material embedded in or associated with the medicament device, wherein said embedded or associated material proportionally changes the magnetic field of the inductive sensor which may be associated with or embedded in the smart device, in one non-limiting example, depending on its distance away from the inductive sensor. The sensor then outputs a variable electrical signal based on the distance between the embedded or associated material and the inductive sensor.

The tactile or vibration component may include a vibration system, which provides a vibratory sensation. In one embodiment, this vibrator system may include a motor, in a non-limiting example a small DC motor, a gear and a weight. The weight may be mounted off-center on the gear, such that when the motor spins, the gear/weight combination at 100 to 150 rpm for example, the off-center mounting creates a vibration. In another embodiment, a linear transducer may be used. In a further example, an eccentric motor can be used to provide an oscillation or vibration.

The terms "medicament training system" and "medicament system" may be used interchangeably herein. The medicament training system or medicament system may be used to train a user to use a medicament delivery device during, before, or after use of the medicament delivery device. A "medicament delivery device" refers to a medicament device that may be used to deliver medication to a user.

The term "medicament device" as used herein may include a training device or a medicament delivery device (i.e., drug delivery device), or a combination thereof. In one non-limiting embodiment, a medicament device may be used to both train a user to use the medicament device and also deliver medicament to the user. In a particular non-limiting embodiment, a medicament device may include different modes, wherein one mode may be used for training only, another may be used for medicament delivery only, and a further mode may be used for training or guidance during medicament delivery, for example. The medicament device may include medicament in some non-limiting embodiments. In other non-limiting embodiments, the medicament device may not include medicament. The medicament device may include an injection device such as an auto-injector and/or trainer, an inhaler device and/or respiratory trainer, a bolus injector, a nasal inhaler device, a needless injector, a transdermal patch, an ampoule, a medicine container, a medicine package, a vial, a metered-dose inhaler, a dry powder inhaler, a prefilled syringe, among other medicament devices known to one of ordinary skill in the art. According to the claimed invention, the medicament device includes a housing and a mechanism having one or more mechanical components that move relative to one another to produce an audible output detectable by the microphone of the collateral device. In non-limiting embodiments, the housing may include the mechanism having one or more mechanical components, wherein the one or more mechanical components are associated with the housing and move relative to one another to produce an audible output, and optionally a visual and/or a tactile output, detectable by a detecting component of the collateral device, for example.

The term "associated" or "association," as used herein, includes but is not limited to direct and indirect attachment, adjacent to, in contact with, partially or fully attached to, and/or in close proximity therewith. The term "in conjunction with" as used herein includes but is not limited to synchronously or near synchronous timing, the phrase may also include the timing of outputs, where one output directly follows another output.

Any of the abovementioned outputs by the signal output component can be presented along with or in conjunction with any of the other outputs of the device. For example, a visual and an audio stimulation or feedback may occur at the same time or within the same step of the training to enhance training of the user. Furthermore, the inventors have discovered that a combination of mechanical feedback (kinesthetic) and electronic feedback enhances the learning experience of a user when using the medicament training system.

According to the claimed invention, a medicament system configured to receive and/or communicate information about a medicament device or about a use of the medicament device, or a combination thereof, to a user is provided. Said medicament system comprises:
a medicament device comprising a housing and a mechanism comprising one or more mechanical components that move relative to one another to produce a mechanical sound during use;
a collateral device to provide information to a user, said collateral device comprising an information detecting and/or receiving component and a collateral device housing comprising an opening for receiving the medicament device, wherein the information detecting and/or receiving component comprises a microphone configured to receive an audible signal from the medicament device and/or the mechanical sound produced by the mechanism, the collateral device further comprising:
   a signal output component;
   a microprocessor;
   a power source; and
   a sensor that detects contact of the collateral device against the skin of a user;
   wherein said collateral device is configured to detect an error condition of the medicament device in response to information received by the microphone or information received by the microphone and information sensed by the sensor, wherein the error condition is incorrect positioning between the device and the user, incorrect contact between the device and the user, an out of order step and/or a step that exceeds or fails to meet a predetermined time value; and
   wherein the signal output component provides an output comprising information about the medicament system and/or a usage of the medicament device and/or medicament system to a user of the system.

In one embodiment, the collateral device includes a sending component configured to send information to the medicament device, and the medicament device may be configured to generate information detectable by the collateral device, or transmit information to the collateral device, wherein the collateral device may be configured to detect and/or receive information about the medicament device from the medicament device and provide information about the medicament device and/or a feedback about a use of the medicament device to a user of the system. The medicament device may further include a transmitter configured to communicate information and/or signals from the medicament device to the collateral device and/or a remote device, and/or receive information and/or signals from a collateral device and/or a remote device.

In a further embodiment, the collateral device of the system may be able to receive or obtain information from a remote source as well as send information to a remote source such as the Internet, a database, a telephone, a computer, or other source, in non-limiting embodiments. The information and/or signal sent or received may include information about the medicament device, a user, a medicament, a use of the medicament device, instructions for using the medicament device or the medicament, a physician or other healthcare provider, a hospital, a pharmacy, in non-limiting examples. In a non-limiting embodiment, the collateral device may include a Smartphone which will be described in more detail below.

The signal output component may be configured to output an audio, a visual, a tactile, a gustatory, or an olfactory signal, or a combination thereof, to the user, in non-limiting embodiments.

According to the claimed invention, the collateral device includes the power source. According to the claimed invention, the medicament device includes a mechanism comprising one or more mechanical parts or components that may move relative to one another to produce a mechanical sound during use, and the information detecting and/or receiving component of the collateral device comprises a microphone. The microphone is configured to receive an audible signal from the medicament device and/or the mechanical sound produced by the mechanism of the mechanical device during use. The microphone detects audio caused by movement of the one or more mechanical parts or components, or movement of components of the medicament device relative to one another by audio detection. The information detected may be provided to a microprocessor and a signal may be provided to a user. Furthermore, the information detected may be stored on a storage medium component. The detecting component may detect movement of the medicament device and/or movement of the mechanical components of the medicament device by visual detection in non-limiting embodiments. In one non-limiting embodiment, the detecting component may include a camera and/or a motion sensor. In a further, non-limiting embodiment, the detecting component may include both an audio and a visual detection component.

In another embodiment, the receiving component may be configured to receive a signal from the medicament device. The signal may provide information about the medicament device, a medicament associated with the device and/or a use of the medicament device.

In a further embodiment, the medicament device may communicatingly connect to the collateral device via a wired or a wireless connection, wherein said connection provides a communication of power and/or information between the medicament device and the collateral device. In some non-limiting embodiments, communication between the medicament device and the collateral device includes one way communication of power and/or information from the collateral device to the medicament device, or from the medicament device to the collateral device, and two-way communication of power and/or information to and from the medicament and collateral devices.

In one non-limiting embodiment, the medicament system is provided wherein the wireless connection includes a Bluetooth ^{®} and/or an RFID (Radio Frequency Identification) technology. In a non-limiting embodiment, where a Bluetooth ^{®} connection is used a power source may be provided in both the medicament device and the collateral device. In a further embodiment, the RFID technology includes an RFID transponder and an RFID reader. In another non-limiting embodiment, the RFID transponder is associated with the collateral device and the RFID reader is associated with the medicament device, the medicament device includes the power source, such that the RFID reader of the medicament device can power the collateral device by way of the RFID transponder.

The RFID transponders, or tags, described herein may be active, semi-active or passive and may include a microchip and an antenna. The active and semi-active RFID transponders may additionally include a battery, in a non-limiting example.

In another non-limiting embodiment, the RFID transponder is associated with the medicament device and the RFID reader is associated with the collateral device wherein the collateral device includes the power source, and wherein the RFID reader of the collateral device powers the medicament device by way of the RFID transponder.

In a further embodiment, the medicament system is provided wherein one of the medicament device or the collateral device comprises a unique identification component. The unique identification component may include, in non-limiting embodiments, information about a medicament, the medicament device, use of the medicament device, or information about the collateral device, or other information know to one of ordinary skill in the art. The other of the medicament device or the collateral device may include a unique identification reader, wherein the unique identification reader is configured to read information on or associated with the unique identification component, in an embodiment. In one non-limiting embodiment, the unique identification component may include a bar code and the unique identification reader may include a bar code reader.

In another embodiment, the medicament system is provided wherein the collateral device includes or is configured to receive pre-programmed information. The pre-programmed information may include information about a medicament device, in a non-limiting embodiment. The pre-programmed information may include information about a medicament, a user, a healthcare provider, a healthcare facility, or other information, in other non-limiting embodiments.

In a further embodiment, the medicament system is provided wherein the collateral device is configured to download or receive information from a database, the Internet, and/or another device. The information may include information about a medicament, a medicament device including instructions for use, contraindication of medicaments, information about a user, medicament dosage information, storage information, prescribing physician information, pharmacy information, manufacturer information, warning information, recall information, in non-limiting embodiments, environmental data, geographical information, time zone information, MET (meteorological) data, storage information, supply chain information, transit data, and/or temperature data. Such temperature data may include, in embodiments in which the medicament device includes medicament, what temperature the medicament has been kept at and/or a report of temperatures that the medicament device and/or medicament has been subjected to from the manufacturer to the user, for example. Other temperature data may include temperature data indicating required or suggested storage temperatures for medicaments and/or medicament devices.

According to the claimed invention, the collateral device includes the signal output component, the microprocessor and the power source. In a non-limiting embodiment, the collateral device may include a storage medium component. In another embodiment, the signal output component may include a speaker, a display, a light, a vibration component, a smell-emitting component, or a temperature-changing component, or a combination thereof.

According to the claimed invention, the medicament system is provided wherein the collateral device is configured to detect an error condition of the medicament device in response to information received by the microphone or information received by the microphone and information sensed by the sensor. According to the claimed invention, the error condition is incorrect positioning between the device and the user, incorrect contact between the device and the user, an out of order step and/or a step that exceeds or fails to meet a predetermined time value. In another embodiment, the medicament system may provide a feedback to a user of the system via the signal output component.

In another embodiment, the medicament system may provide instructions for using the medicament device in a sequence of steps, wherein the microprocessor is configured to control a provision of the instructions for using the medicament device to the user in the sequence of steps and/or to provide an instruction to the user based on information detected and/or received by the information detecting and/or receiving component of the collateral device.

In a further embodiment, the system may include a sensor associated with the medicament device, wherein the sensor may be configured to detect a condition of the medicament device. Information about the condition detected by the sensor may be communicated to the collateral device and/or to a user from the medicament device, in an embodiment. The information that is communicated to the collateral device may further be provided from the collateral device to the user in a non-limiting embodiment.

In yet another embodiment, the medicament system includes an attachment component configured to secure the collateral device to the medicament device. The system may further include in an embodiment, a sending component configured to send information to the medicament device and/or the collateral device

In a non-limiting embodiment of the medicament system, the collateral device may include the microprocessor and the storage module, the collateral device including pre-programmed information about one or more medicament devices and/or instructions for use for one or more medicament device and/or medicaments associated therewith, for example.

According to the claimed invention, the medicament device comprises a mechanism comprising one or more mechanical components that move relative to one another to produce a mechanical sound during use. In an embodiment of the medicament system, the one or more mechanical components are associated with the housing and move relative to one another to produce an audible, and optionally a visual and/or a tactile, output detectable by the detecting component. In one non-limiting example, wherein the medicament device includes a respiratory device or trainer and specifically a metered dose inhaler, for example, the one or more mechanical components of the medicament device may include the components of the metered dose inhaler housing that move relative to one another when the inhaler is actuated.

According to the claimed invention, the collateral device is configured to detect an error condition of the medicament device in response to information received by the microphone or information received by the microphone and information sensed by the one or more sensors. The collateral device detects movement of or a condition of the medicament device by way of the microphone which listens for sounds generated by the movement(s) of the medicament device and/or its components to indicate, for example, the cap has been removed, a safety shield has been depressed in preparation for an injection with an injection device, in non-limiting embodiments. In one non-limiting example, the collateral device may detect movement of the components of the medicament device by way of a camera, for example, such as detecting the movement of the mechanical components of a metered dose inhaler device when the metered dose inhaler device is actuated, for example.

In still a further embodiment, the system may provide instructions for using the medicament device in a sequence of steps, wherein the microprocessor is configured to control a provision of the instructions for using the medicament device to the user in the sequence of steps and/or to provide an instruction to the user based on information received from the sensor of the medicament device.

In a further embodiment, the attachment component may include a hook, a clip, an adhesive, a holder which may be contoured to receive the medicament device in a non-limiting embodiment, a magnet, or a combination thereof, or any other type of attachment component known to one of ordinary skill in the art. In one non-limiting embodiment, an attachment component may be formed by a portion of the housing of the collateral device, the attachment component may include a surface of the housing, wherein the surface is complementary in shape to the portion of the medicament device upon which it is to be attached, therefore, in one non-limiting example, the attachment component may be snapped onto the medicament device by contact with the surface of the housing of the medicament device and the complementary surface of the housing of the collateral device. In a non-limiting embodiment, the attachment component may be attached to one of the medicament device or the collateral device, and configured to attach the medicament device or the collateral device to the other of the medicament device or the collateral device. For example, the attachment component may be affixed to the collateral device and configure to attach the collateral device to the medicament device, in another non-limiting example, the attachment component may be affixed to the medicament device, and be configured to attach the medicament device to the collateral device. In a further non-limiting example, the attachment component may be a component separate from the medicament device and the collateral device, but may be configure to attach to both of the medicament device and the collateral device.

In the embodiments provided herein, the medicament system embodiments may further comprise a control interface, wherein the control interface may include a responsive member reactive to user input. The control interface may be associated with the medicament device and/or the collateral device in non-limiting embodiments.

Furthermore, as aforementioned herein, the medicament device embodiments described herein may include a medicament training device, a medicament delivery device, or a combination thereof.

According to the claimed invention, the collateral device includes a sensor that detects contact of the collateral device against the skin of a user. The sensor may be associated with the collateral device housing, in one embodiment. In a further embodiment, the collateral device may include a proximity sensor, a perpendicularity sensor, a contact sensor, a temperature sensor, a motion sensor, a light sensor, a fluid flow rate sensor, a strain gauge sensor, a pressure sensor, a tilt sensor, a force sensor, a level sensor, a photoelectric sensor, a magnetic sensor, an ultrasonic sensor, an electrochemical sensor, an acceleration sensor, a moisture sensor, a humidity sensor, a speed sensor, an inductive sensor, a capacitive sensor, or an orientation sensor, or a combination thereof, in a non-limiting embodiment.

The collateral device includes a housing having an opening for receiving the medicament device. The collateral device includes a sensor (also denoted "first sensor"). The first sensor may be on a bottom surface of the collateral device. In another, non-limiting embodiment, the first sensor may form a portion of the bottom of the collateral device. In one embodiment, this first sensor may include a capacitive sensor, for example. The first sensor senses when the collateral device is against the skin of a user. The first sensor may additionally sense when a contact between the safety shield and the first sensor has been made.

In one, non-limiting embodiment, the housing of the collateral device has a top portion, and a bottom portion. In this particular embodiment, the collateral device may include a second sensor configured to protrude into the opening, wherein a contact between the medicament device and the second sensor indicates that a cap of the medicament device has been removed before placement into the collateral device, as a result of the shape of the distal end of the housing of the medicament device with the cap removed, and the resulting contact between this portion of the medicament device and the second sensor. Therefore, insertion of the medicament device into the collateral device causes information to be detected via the second sensor that the cap has been removed from the medicament device prior to insertion, resulting in activation of the second sensor as the second sensor contacts the medicament device. Further insertion of the medicament device into the collateral device causes the second sensor to move from a first position to a second position, further activating the second sensor as it is moved from the first to the second position as the medicament device is further inserted into the collateral device. In a non-limiting embodiment, the second sensor may include a mechanical switch, or contact switch.

A third sensor may be provided in near, or adjacent to the opening of the collateral device, wherein the third sensor is configured to detect a position of a needle shield of the medicament device. Further insertion of the medicament device into the collateral device causes the needle shield to contact the bottom portion of the collateral device, movement of the medicament device against the bottom portion of the collateral device causes retraction of the needle shield enabling actuation of the medicament device. The third sensor, in a non-limiting embodiment, may contact the medicament device when the needle shield is in a retracted position. This may be due to the placement of the third sensor as well as the tapered shape of the distal end of the medicament device housing, such that the medicament device housing only contacts the third sensor when the needle shield has been fully retracted. The third sensor may include a contact sensor or a contact switch in non-limiting embodiments. Therefore, activation of the second and third sensors may indicate that the medicament device is activated and ready for use. Feedback may be provided to a user via the medicament device or the collateral device during the interaction between the medicament device and the collateral device before and after the medicament device is activated for use. In one non-limiting embodiment, the collateral device may include an aperture in the bottom portion to allow an injection member to traverse the bottom portion of the collateral device during use of the device. In another embodiment, no aperture is provided.

Either alone, or in combination with the third sensor, the first sensor may indicate to a user that the medicament device has been fully inserted into the collateral device, the collateral device is against the skin of the user and ready for use, and the safety shield has been retracted into the housing of the medicament device. A combination between contact of the third sensor with the medicament device distal end and contact of the first sensor with the medicament device would indicate the shield has been fully depressed (i.e., retracted), and the injection device is ready for activation.

In a further embodiment, the collateral device may include an internal element that is movable relative to the collateral device. The internal element may include one or more sensors, and may include an opening configured to receive a portion of the medicament device. The internal element may be disposed within the opening of the collateral device, and may receive the medicament device, and be displaced upon movement of the medicament device relative to the collateral device. The collateral device may be placed against the skin of the user, the medicament device distal end may be placed within the opening of the internal element, a spring may be provided to allow displacement and replacement of the internal element relative to the collateral device. Movement of the medicament device into the internal element opening causes activation of contact sensors to indicate removal of the cap of the medicament device and correct placement within the opening, additional sensors may sense contact between the collateral device or the internal element with the skin and with the distal end of the medicament device or the safety shield of the medicament device. These sensors may be provided, as described herein, to detect contact with the medicament device, and depression of the safety shield in preparation for use of the medicament device. Upon movement of the medicament device relative to the collateral device, the internal element is displaced from the collateral device into the skin of the user, the safety shield of the medicament device is depressed, and the medicament device may be actuated. The internal element may include an opening to allow for passage of the injection member of the medicament device to enter a target area on the user skin to deliver an injection. Following the injection, and release of any pressure on the medicament device, the biasing member may extend to retract the internal element into the collateral housing.

In other embodiments described herein, the collateral device may include an attachment component, which may be inherent in the shape and size of a portion of the collateral device, such that it mates with a complementary shape and size of the medicament device, so as to attach onto the medicament device in one, non-limiting embodiment. In other non-limiting embodiments, the attachment component may include an adhesive, a clip, or any other type of attachment component described herein or known to those skilled in the art to attach the collateral device onto the medicament device. In one embodiment, the collateral device may include a contact element and contact switch, wherein upon movement of the contact element relative to the collateral device, the contact element makes contact with the contact switch to register a status of the medicament device, such as, for example, depression of the safety shield of the device in preparation for an injection. Due to the position of the contact element when the collateral device is attached onto the medicament device, in this non-limiting embodiment, the interaction between the contact element and the contact switch registers that the shield has been deflected when the contact element presses against the contact switch, for example.

In a further embodiment, the collateral device is provided wherein the storage module includes pre-programmed information. The pre-programmed information may include information about a medicament device and/or instructions for use for a medicament device and/or a medicament associated therewith, in a non-limiting example. The collateral device may be further configured to receive or download information about a medicament, a medicament device, and/or a user from the Internet, from another device, and/or from a database, wherein the information can be received or downloaded via a wired and/or a wireless connection.

In still a further embodiment, the collateral device may be configured to provide instructions and/or a feedback to the user via the signal output component.

In a further embodiment, the collateral device may be configured to communicate with, a medicament delivery device, a medicament training device, or a combination thereof. The collateral device may communicate with the medicament device(s) by receiving and/or sending information, receiving and/or sending signal(s), receiving and/or sending power, etc., in non-limiting examples.

In a further embodiment, the collateral device may provide instructions for using the medicament device in a sequence of steps, and wherein the microprocessor is configured to control a provision of the instructions for using the medicament device to the user in the sequence of steps and/or to provide an instruction to the user based on information produced by or detected by the sensor of the medicament device. Error conditions may include, in non-limiting examples, putting cap back on a device, an out of sequence operation of a device, a wet injection, and not holding at 90 degrees when required.

In an embodiment, the collateral device may include a control interface; the control interface may include a responsive member reactive to a user input.

A method of using a collateral device to train a user of a medicament device to properly operate the medicament device to dispense a dose of medicament and to provide an instruction and/or a feedback to a user of the collateral device is provided. The method may include detecting and/or receiving information from a medicament device, processing information received from the medicament device, and providing a signal output to a user based on information received and processed.

The method may include providing instruction and/or information related to a medicament device, a medicament, and/or a user to a user, wherein the instruction and/or information is stored on a storage module associated with the collateral device. The method may further include providing instruction and/or information related to a medicament device, a medicament, and/or a user to a user, wherein the instruction and/or information is received by the collateral device via a wired and/or a wireless connection.

The method may include electronically reading information from a unique identification component associated with the medicament device and/or a medicament, wherein the information is output to a user.

In another embodiment, a collateral device configured to receive information from and/or detect information about a medicament device is provided, wherein the collateral device includes a collateral device housing, a sensor associated with the housing; and an attachment component associated with the housing, wherein the attachment component is configured to attach the collateral device to the medicament device.

In the embodiments described herein, the collateral device may include a Smartphone, a computer, a PDA, or the like. These devices may include the microprocessor, information detecting component and receiving component, signal output component, storage medium, power source, or a combination thereof, such that a signal or information from a medicament device can be received by a Smartphone device, in a non-limiting example, and the Smartphone may provide the feedback or output to the user based on the information or signal received. A display of the Smartphone may be beneficial to provide feedback to a user based on information received from the medicament device or received from a remote source such as the Internet, a database, another phone, or a remote computer, in non-limiting examples. In these non-limiting embodiments, it is beneficial to take advantage of the components already existing in a Smartphone (or other similar device), to provide training or guidance to a user during use of the medicament device.

Embodiments of the system and medical device described herein may include sensors to provide users with an active learning experience. If an error is made in the training sequences, patients are notified through spoken instructions and taught how to overcome the error in non-limiting embodiments. If the user makes a mistake, in a non-limiting embodiment, the training injector may provide spoken guidance and encouragement to help establish synchronous motor skills to prevent errors in the future.

In another non-limiting embodiment, the wireless communication between the medicament device and the collateral device may include Radio Frequency Identification technology, for example. According to the claimed invention, the collateral device includes a power source. In an example, the collateral device may include an RFID reader and the medicament device may include an RFID transponder, wherein the RFID transponder may be powered by the RFID reader.

In a non-limiting embodiment, the collateral device may be preprogrammed to interact with various configurations of medicament devices. Medicament devices may include inhalation devices or inhalation training devices, injection devices including autoinjectors, pre-filled syringes or any other devices for parenteral administration of medicament (or related training thereof), devices including medicament bottles or tubes housing medicament (such as, for example, a pill bottle containing capsules or tablets or a vial containing medicament in non-limiting examples). The collateral device can be configured to associate with one or more medicament devices and one or more medicament training containers, in non-limiting embodiments. Information can be communicated between devices of the system by means known in the art, communication may occur between the medicament device and the collateral device and optionally between the medicament device and/or the collateral device and a remote device.

Information communicated between devices may include, for example, compliance information including information about the usage of the system or device. Additional information may include previous uses of the medicament device and/or system, correct and incorrect usage of the device and/or system as well as instructions for use of various medicament devices, contraindications related to various medicament devices and various medicaments, possible medicament interactions, safety information and storage information, reminders or timers identifying next scheduled administration of medicament, dosage information, volume and/or strength of medicament, other instructions regarding medicament including, but not limited to compliance to therapy or treatment, warnings, and any other instructions, among other information typically associated with medicaments and medicament devices. Information may further be communicated between the collateral device and a remote device, such as, for example, a Smartphone, a computer, a database, a PDA, a digital or analog watch, other remote device, or other information receiving device. Information communicated from the collateral device may include information about a use of the medicament device, in a non-limiting embodiment. The remote device may be adapted to receive the information and/or process the information and provide a feedback to a user of the system or an additional user such as a medical professional, a family member, or other person or entity. Information may be communicated between the collateral device and one or more remote devices, between the collateral device and the medicament device, or between the medicament system or medicament training device and one or more remote devices. Information can be communicated, as described herein, by way of wired and/or wireless communication. Feedback provided by the remote device may include training information, information about a correct or incorrect usage of the device, system, or medicament, error correction information, positive feedback, reminders regarding using the device, system, or taking a medicament. Other reminders may be provided by the remote device including, but not limited to reminders to make an appointment with a physician, reminders to refill a prescription, reminders to take a medicament, reminders to train using the medicament device, collateral device and/or system, among other reminders.

Non-limiting examples of collateral devices described herein may include a Smartphone, a non-electronic device, other types of electronic devices including, but not limited to, a computer, a PDA, a tablet, a digital or analog watch, for example.

In additional embodiments of the systems and devices provided herein, an intermediate collateral device may be provided, wherein the intermediate collateral device may associate with, or in a non-limiting embodiment, be attached to the medicament device. The intermediate collateral device may include a sensor and/or a transmitter in a non-limiting embodiment. The intermediate collateral device may be configured to communicate (i.e., communicatingly connects) either wirelessly or by wired connection with the medicament device, a collateral device, and/or a remote device. The communication may include one-way communication to or from the intermediate collateral device, or two-way communication there between. The communication may include a communication of power or information, including a communication of signals there between. In alternative non-limiting embodiments, the intermediate collateral device may be attached to a collateral device or a remote device.

Embodiments of the systems and devices disclosed herein may further include a remote communication component, wherein the remote communication component is configured to provide communication between the medicament system and/or the user and a remote source. The communication component may be used by a remote source (or entity) to contact the system and/or the user of the system or retrieve information therefrom or send information thereto. The communication component may also, or alternatively be used by the user of the system to access a remote source (or entity) to communicate therewith, receive information from and/or send information to. In non-limiting embodiments, the remote source may include emergency personnel or other healthcare professional, a pharmacy personnel, a help personnel or system operator who may be able to answer questions regarding the device or system or receive information there from, a computer or central network accessible for help using the system, for example, and/or a family member or other person, for example. The remote communication component may provide access to the user via the system to a remote source and/or provide remote source access to the system and/or the user.

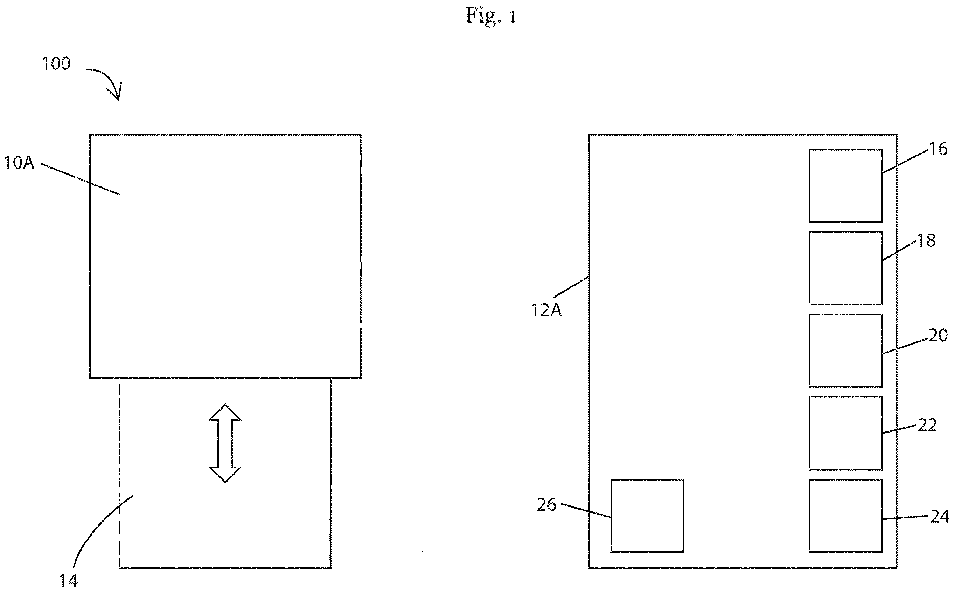

Non-limiting embodiments of the system and device are provided in the Figures described herein. FIG. 1 provides a schematic view of a medicament system 100 comprising a medicament device 10a and a collateral device 12a. The medicament device 10a comprises a housing 14, which includes one or more mechanical components that may move relative to one another during the use of the medicament device 10a. For example, where the medicament device 10a includes a respiratory inhaler device or trainer, there may be one or more mechanical components of the device that when moved relative to one another during use of the device makes signature clicking sounds. These clicking sounds can be observed by a user and may be indicative as to whether the device is being used correctly or incorrectly.

The collateral device 12a of FIG. 1 further includes an information detecting component 16, an information sending and/or information receiving component 18 (the sending and receiving components may be two separate components in other non-limiting embodiments), a microprocessor 20, a storage medium component 22, a power source 24, and a signal output component 26. The information receiving component 18 and/or the information detecting component 16 of the collateral device 12a comprises a microphone and is able to receive signals or information from and/or detect movement of the medicament device 10a, or components thereof. This information may be used to determine whether the medicament device is being used, whether it is being used correctly, and to provide any other information about the medicament device and/or from the medicament device 10a to the collateral device 12a. The information detecting component 16 may include a camera, in non-limiting examples.

The information sending component of the collateral device 12a may be used to send information or signals to the medicament device 10a, in a non-limiting embodiment. The signal output component 26 of the collateral device 12a may be used to provide information and feedback to a user of the system 100, in a further embodiment. This feedback may be provided by any means known in the art, including but not limited to, by visual stimuli, audio, vibration, temperature change, gustatory and other types of feedback may also be used. Consequently, the signal output component may include a light or display, or a combination thereof, and/or a speaker, in non-limiting embodiments. The storage medium component 22 may be used to store information including instructions for using the medicament device 10a in an embodiment, and more specifically, stepwise instructions in a further embodiment. The storage medium component 22 may further include information about, instructions for use of multiple medicament devices among other information stored thereon. This information may be stored on the storage medium component 22 and may be pre-loaded and/or retrieved by the system 100 in non limiting embodiments.

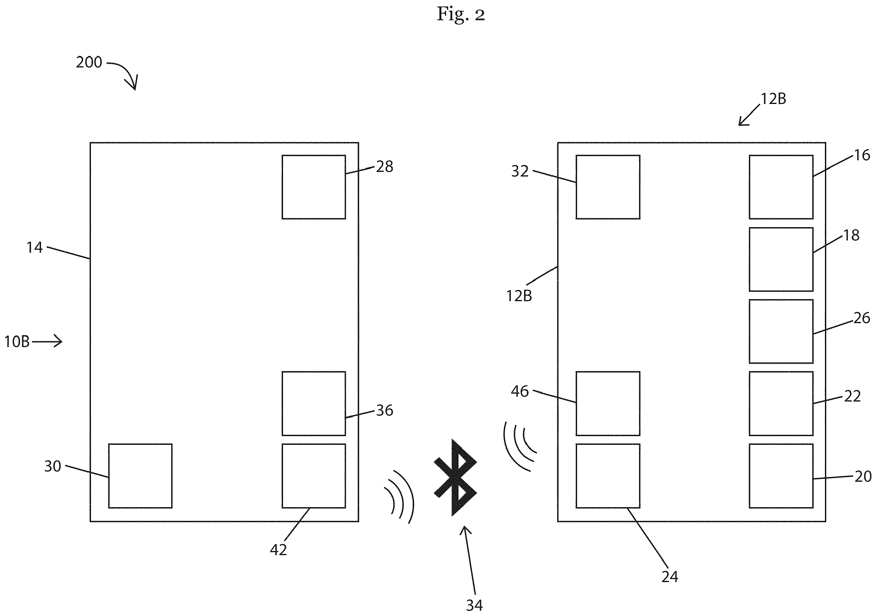

In another embodiment, a medicament system 200 is shown in the schematic view of FIG. 2, including a medicament device 10b having a housing 14, a collateral device 12b communicatingly connected to the medicament device via wired or wireless connection (wireless RFID connection shown in FIG. 2). The wireless RFID (Radio Frequency Identification) connection is shown in FIG. 2, wherein the RFID transponder 30 is associated with the medicament device 10b, and the RFID reader 32 is associated with the collateral device, however, in other embodiments the RFID components may be associated with other devices of the system and/or the RFID transponder 30 may be associated with the collateral device 12b and the reader 32 associated with the medicament device 10b, in non-limiting examples. Information and signals may be communicated between the RFID transponder 30 and the RFID reader 32 between the devices of the system 200 as described herein. Power can also be communicated or transmitted there between as aforementioned. Communication of information, signals and/or power may include two -way communication or one way communication from one component to another component of the system 200 in non-limiting embodiments, as described above. Communication of information can also or alternatively be provided by way of Bluetooth connection 34 as shown in the non-limiting embodiment of FIG. 2. The medicament device 10b may further include a sensor 28, and a transmitter 36 to send a signal from sensor inputs, for example, to the collateral device 12b. The collateral device 12b includes an information detecting and/or receiving component, a signal output component 26, a microprocessor 20, and a power source. The collateral device 12b may include a storage medium component 22, in non-limiting embodiments. The collateral device may also include an information sending component to send information to the medicament device 10b, in a non-limiting embodiment.

Embodiments of the system described herein, such as, in a non-limiting example, the system of FIG. 2 provide communication between a medicament device 10b and a collateral device 12b, wherein the collateral device may include a Smartphone or other such device which may include some of the various components described.

Embodiments of the medicament system provided herein, for example, as in FIG. 2 may be used to train a user to use the medicament device, wherein feedback about a use of the device, about a medicament, or about a user, for example, may be provided by the collateral device. Furthermore, the medicament system embodiments may be used to guide a user to deliver medicament or train a user to use a medicament device, wherein the user is a training-only or training and deliver device, by providing feedback to the user on the collateral device 12b, in a non-limiting embodiment. Consequently the connection between the medicament device 10b and the collateral device 12b can provide a communication of information such that the collateral device 12b may be used to communicate information about the medicament device 10b or a user thereof to the user, in non-limiting embodiments.

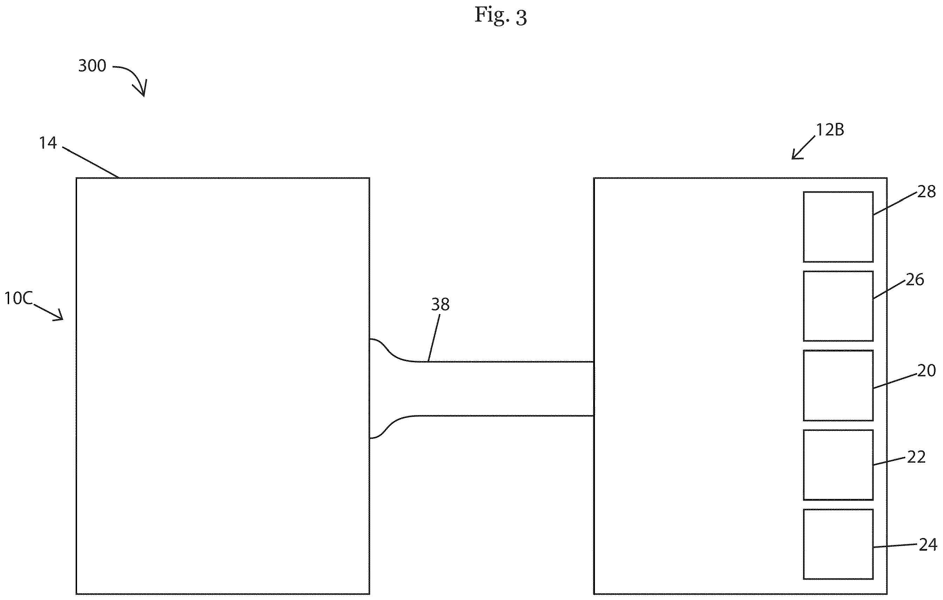

In a further embodiment, a medicament system embodiment 300 is provided in FIG. 3, wherein a medicament device 10c including a housing 14 is configured to be attached to a collateral device 12c (or vice versa, the collateral device 12c may be configured to be attached to a medicament device 10c) by way of an attachment component 38, in a non-limiting embodiment. The collateral device 12c may include a storage medium component 22. The collateral device 12c includes a sensor 28, a signal output component 26 to communicate and provide feedback to a user, a microprocessor 20 to process signals received from the sensor 28, and a power source 24 to provide power to the collateral device 12c and, in further non-limiting embodiments, to the medicament device 10c by way of wired or wireless connections as described herein. In the medicament system embodiment 300, the medicament device 10c may have, but need not include any components, wherein all the system components may be in the collateral device 12c. Information can be sensed by and/or received by the collateral device 12c from the medicament device 10c (and in some embodiment sent to the medicament device 10c from the collateral device 12c), via the attachment between the collateral device 12c and medicament device 10c through the attachment component 38.

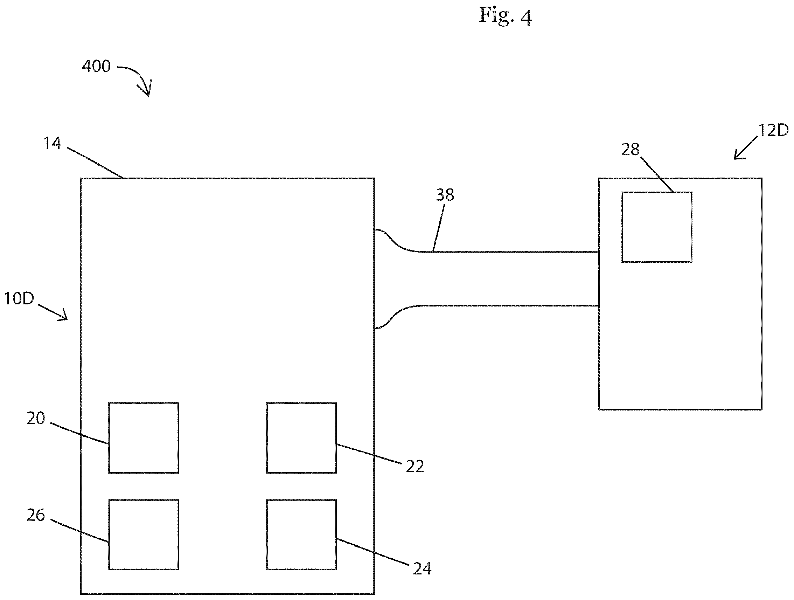

In a not-claimed example of the medicament system 400 shown in FIG. 4, the medicament device 10d and the collateral device 12d may be configured to be attached to one another by way of an attachment component 38. The collateral device 12d may include a sensor 28, and the medicament device may include a housing 14, a microprocessor 20, a storage medium component 22, power source 24, and signal output component 26. In this embodiment the power source may be used to power the collateral device 12d by way of a wired or wireless connection between the medicament device 10d and collateral device 12d.

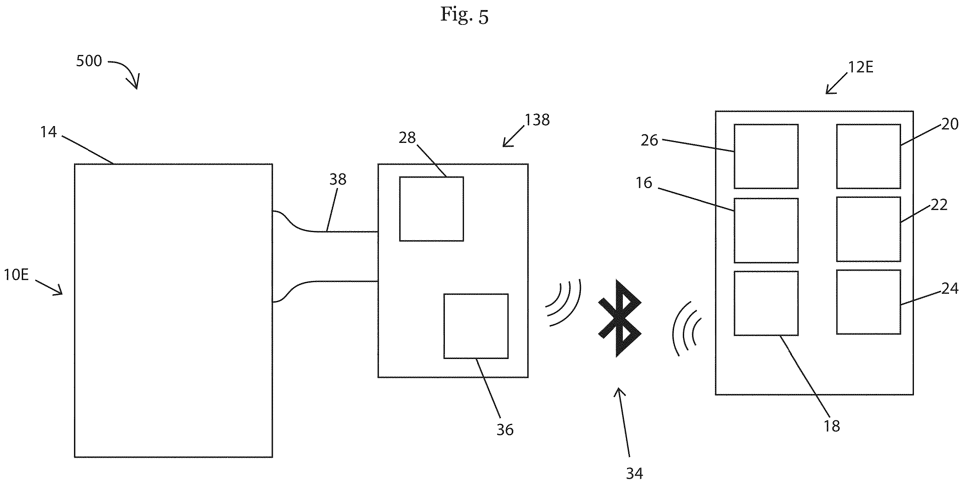

In yet a further embodiment of the medicament system 500 shown in the schematic of FIG. 5, a medicament device 10e having a housing 14 and an intermediate collateral device 138 are configured to be attached to one another by way of an attachment component 38. The intermediate collateral device 138 may include a sensor 28 to sense information about the intermediate collateral device 138 and therefore, the medicament device 10e by way of its attachment thereto. Sensor information can include location information, position, orientation, among other information as described herein that can be sensed by one or more sensors of the system. The medicament system embodiment 500 further includes a collateral device 12e, which includes the signal output component 26, the information detecting component 16, information receiving component and/or sending component 18, microprocessor 20, and power source 24 and which may include other components of the system such as, for example, storage module 22, in a non-limiting embodiment. The collateral device 12e may be associated with the intermediate collateral device 138 by way of a wired and/or a wireless connection as described herein, for example, by a Bluetooth^{®} connection 34. Furthermore, in a further non-limiting embodiment, the collateral device 12e may connect wirelessly or by wired connection with the medicament device 10e.

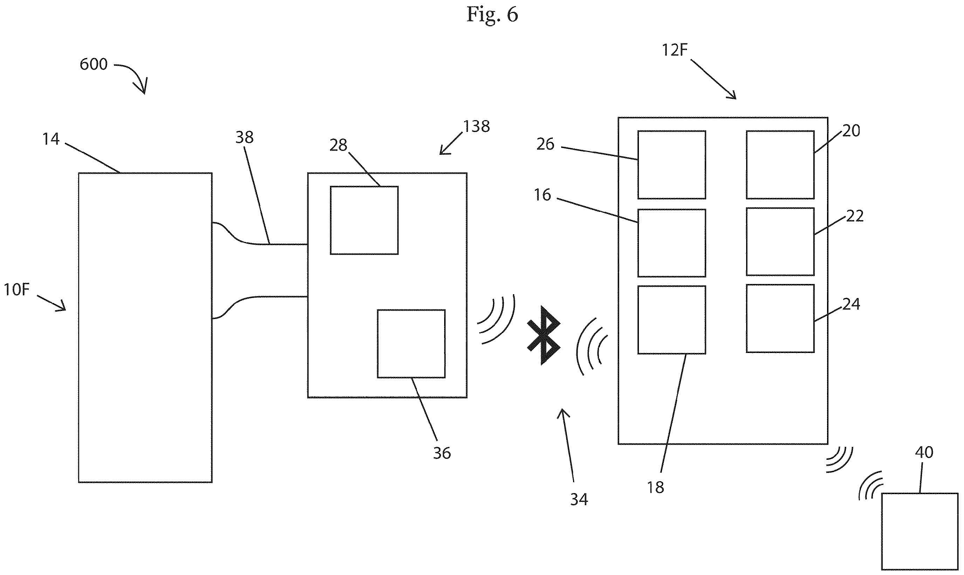

In yet a further embodiment of the medicament system 600 shown in FIG. 6, a medicament device 10f may be associated with an intermediate collateral device 138. The association between the medicament device 10f and the intermediate collateral device 138 may include a wired or a wireless connection, or may include an attachment component 38 as shown in FIG. 6. The intermediate collateral device 138 may further be communicatingly connected to a collateral device 12f. The collateral device 12f may be further communicatingly connected to a remote device 40. The connections between the devices of the system 600 may include wired or wireless connection as described herein, the wireless connection including but not limited to RFID or Bluetooth^{®} connection. The remote device 40 may include a database to which information can be uploaded to from the system 600 or downloaded from to the system 600. The remote device 40 may further include another device, a computer, a telephone, a smart device, or a watch, among other devices. These devices may allow other persons or entities to send information to the system 600, retrieve information from or receive information from the system 600, in non-limiting embodiments. Additionally, in the embodiment of 600, the intermediate collateral device 138 or the medicament device 10f may be configured so as to send information to or receive information from the remote device 40, by either wired or wireless communication as discussed herein.

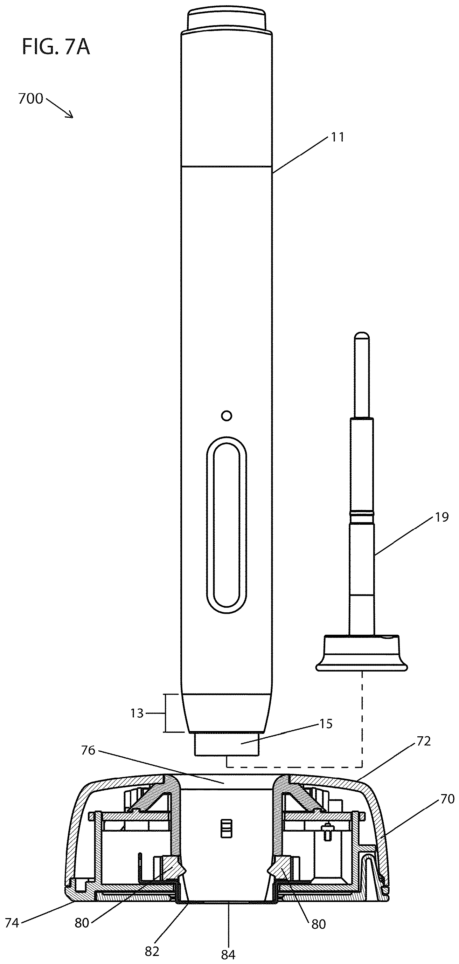

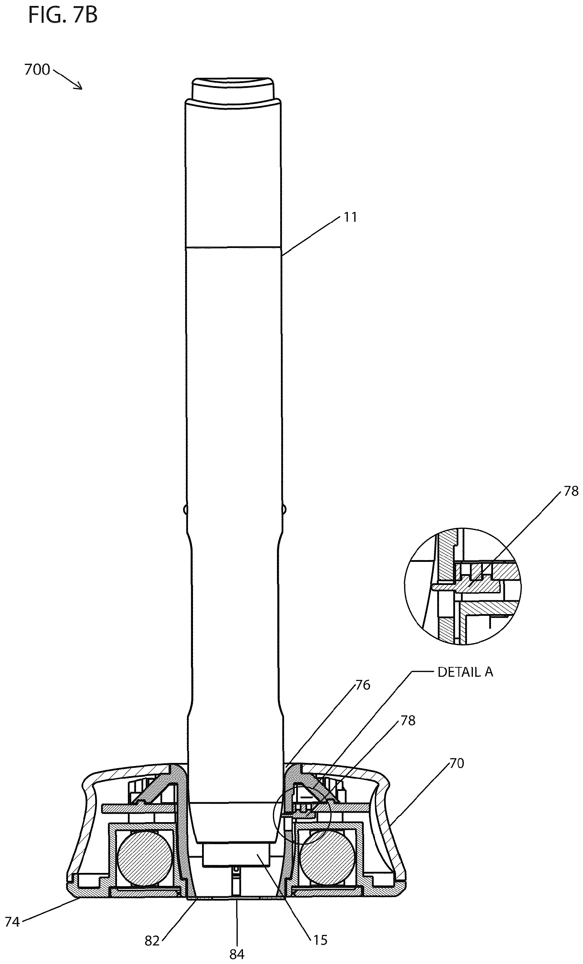

FIG. 7A is a side view of an embodiment of a medicament system 700 configured to receive and/or communicate information about the system, about a medicament device, or about a use of the medicament device, or a combination thereof to a user. The medicament system 700 includes a medicament device 11 having a housing, a removable cap 19, a distal end 13, and a retractable safety shield 15. In one non-limiting embodiment, the distal end 13 may include a tapered shape as shown in FIG. 7A. The system 700 further includes a collateral device 70, shown in cross-sectional view in FIG. 7A, including a top portion 72, a bottom portion 74, an opening 76 for receiving a portion of the medicament device 11, at least one sensor 80, 82, an injection member aperture 84. FIG. 7A shows the medicament device 11 with the cap removed 19 prior to insertion into the opening 76. In the embodiment 700 shown in FIG. 7A, the sensor 82 forms a portion of the bottom portion of the collateral device. The sensor 82 is a capacitive sensor, embodied as a plate, and configured to detect placement of the collateral device against the skin of a user, and optionally detect contact with a portion of the medicament device 11 once fully inserted into the collateral device 70 (i.e., to detect contact with the safety shield or distal end 13 of the medicament device housing). As shown in the side view of FIG. 7B, the medicament device 11 is further inserted into the opening 76 of the collateral device 70. FIG. 7B shows a cross-sectional view of the collateral device from a point of view rotated approximately 90 degrees from the view seen in FIG. 7A.

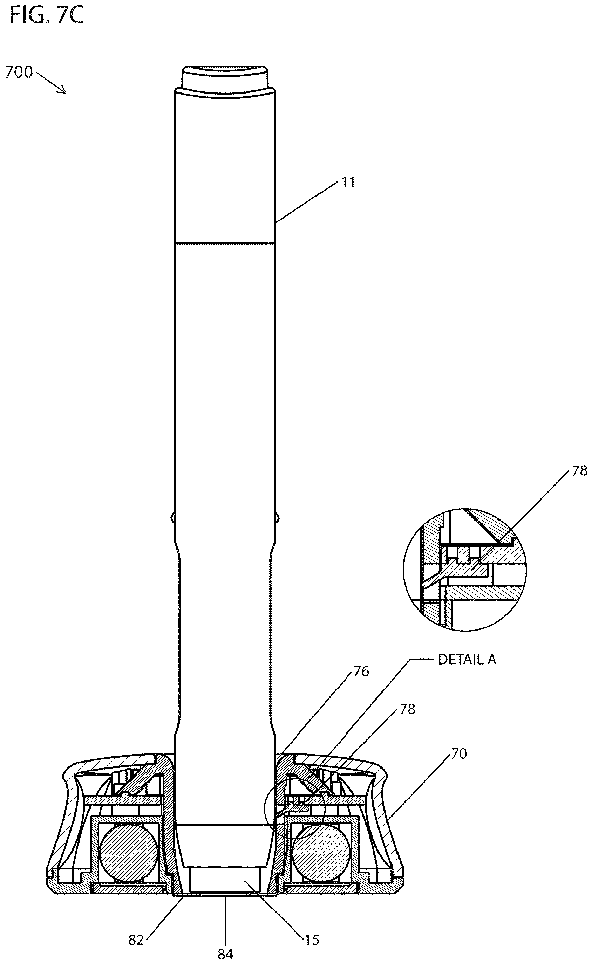

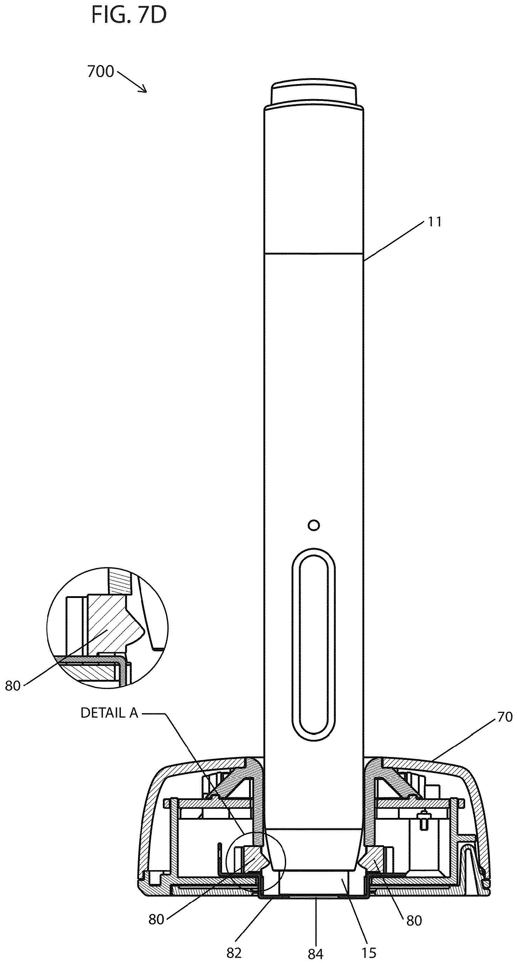

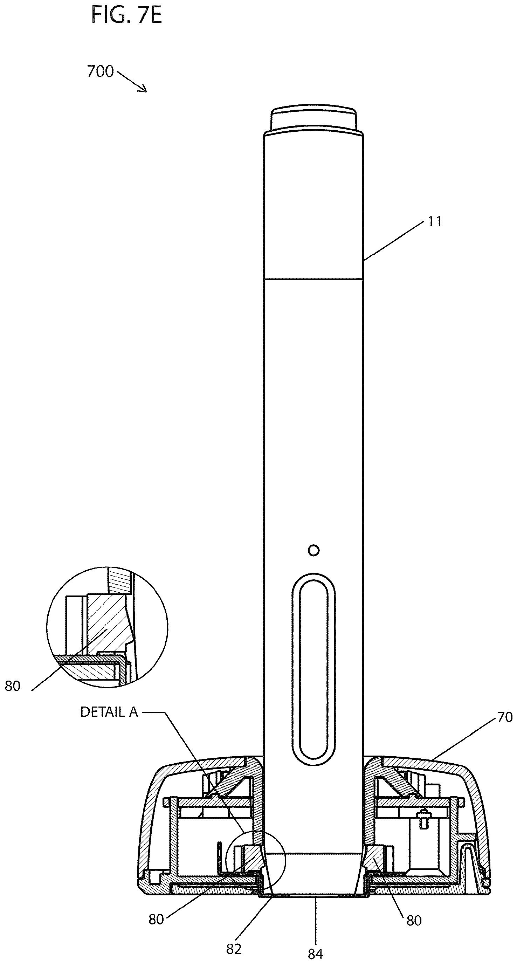

The view of FIG. 7B shows another sensor 78 of the collateral device, adjacent to the opening 76, wherein the sensor 78 detects insertion of the medicament device without cap 19, due to the contact between the sensor and the distal end of the medicament device 11. If the medicament device 11 were inserted with cap 19 on the device, there would not be contact between the sensor 78 and the distal end of the medicament device 11, and therefore, the sensor 78 would not sense contact with the medicament device 78. This may produce an error in the system, for example, which may be output via a signal output component shown in FIG. 7G, or no indication to a user at all, in non-limiting examples. Upon proper removal of the cap 19 and insertion of the medicament device 11 into the opening 76 as shown in FIG. 7B, contact is made with the sensor 78, and a signal output may be provided to a user. Sensor 82 senses contact between the collateral device and the skin of a user, and a signal output may be provided to indicate the status of the system. Further insertion of the medicament device 11 into the opening 76 of the collateral device 70, as shown in FIG. 7C, shows movement of the sensor 78 (i.e., a mechanical switch in one non-limiting embodiment as shown) to a second position, as the medicament device safety shield 15 reaches the bottom portion of the collateral device 70 housing. The injection member aperture 84 is shown in FIG. 7C. FIG. 7D shows a 90 degree rotation of the collateral device, wherein the sensors 80 for sensing depression of the safety shield (shown in FIG. 7A), are not yet in contact with the distal end 13 of the medicament device 11. The injection member aperture 84 within the sensor 82, which forms part of the lower portion of the collateral device is shown. The needle shield 15 is not yet depressed. FIG. 7 E provides a view of the system 700 shown in FIG. 7D, wherein the medicament device 11 is inserted into the collateral device 70, and the safety shield 15 (not shown) is depressed (i.e., fully retracted within the medicament device 11). The system 700 detects retraction of the safety shield, as the sensor 80 contacts the medicament device 11 as shown in FIG. 7E, activating the sensor 80. Aperture 84 is shown in the sensor (i.e., plate) 82.

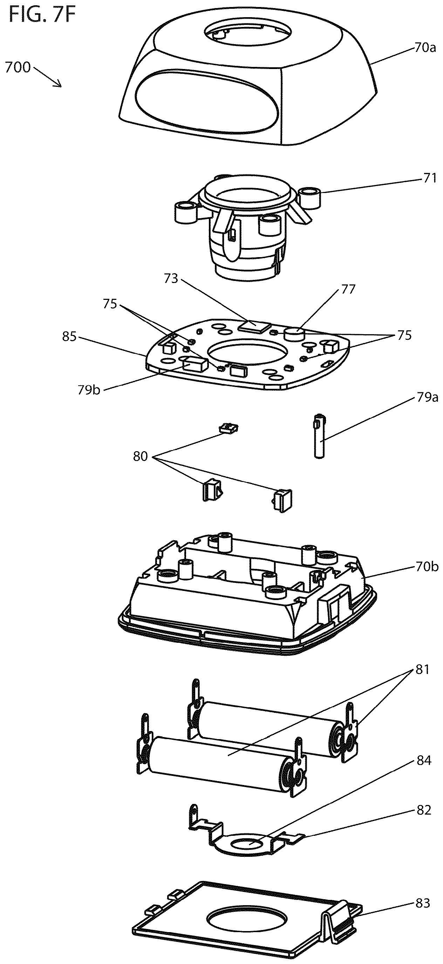

FIG. 7F shows an exploded view of the components of the collateral device 70 of the embodiment 700. A top housing portion 70a of the collateral device housing is provided, as well as a light pipe 71 which is configured to direct light from any light source provided on a printed circuit board assembly 85 located there below. For example, the signal output component 75 may include light (i.e., light emission diode (LED), for example). When the collateral device is assembled, the light from the LEDs 75 may be directed via light pipe 71 to a user of the system. The lights may be provided to a user as a form of feedback before, during or after use of the system, in one non-limiting embodiment. The lights may also provide indication of powering on or off of the system or the collateral device 70, in other non-limiting embodiments. In further non-limiting embodiments, as discussed herein, the signal output component 75 may include lights, and/or an audio component, such as a speaker, for example, to provide audio feedback to a user, any other type of signal output component 75 described herein or known to those skilled in the art.

The printed circuit board assembly 85 may further include the microprocessor 73, the microphone 77 to receive sounds from the medicament device 11, and a blue tooth transmitter 79b in one non-limiting embodiment, which works in conjunction with a blue tooth pairing button 79a used to pair the collateral device to another device. The blue tooth components 79a, 79b may be used to sync the device 70 and/or system to other devices, to phone applications, or to transmit information or receive information to/from the medicament device, other medicament devices, other collateral devices or other transmitting/receiving components or databases. The microphone 77 is used to detect sounds of the medicament device 11 including movement of the medicament device or the components there of relative to one another to indicate a status of the medicament device 11, such as, for example, removal of the cap 19, contact of the safety shield, depression of the safety shield, delivery of the injection member, insertion of the injection member into the target area, delivery of medicament, completion of delivery of medicament, retraction of the injection member, extension of the safety shield, and other such sounds indicating a status of the medicament device 11.

The sensors 80 are shown as contact switches in the exploded view of FIG. 7F, these may also be embodied as other sensor types described herein. The bottom housing 70b of the collateral device is shown, as well as the power source 81 shown as batteries in the embodiment provided herein. Other types of power sources may additionally or alternatively be used. The sensor 82 with injection member aperture 84 is provided. In one non-limiting embodiment, the sensor 82, may include a capacitive contact plate as described herein. As known to those skilled in the art, a capacitive contact sensor or contact plate may determine contact against a surface (i.e., target surface like the skin of a user), by detecting resistance. A power source housing cover 83 is shown in FIG. 7F.

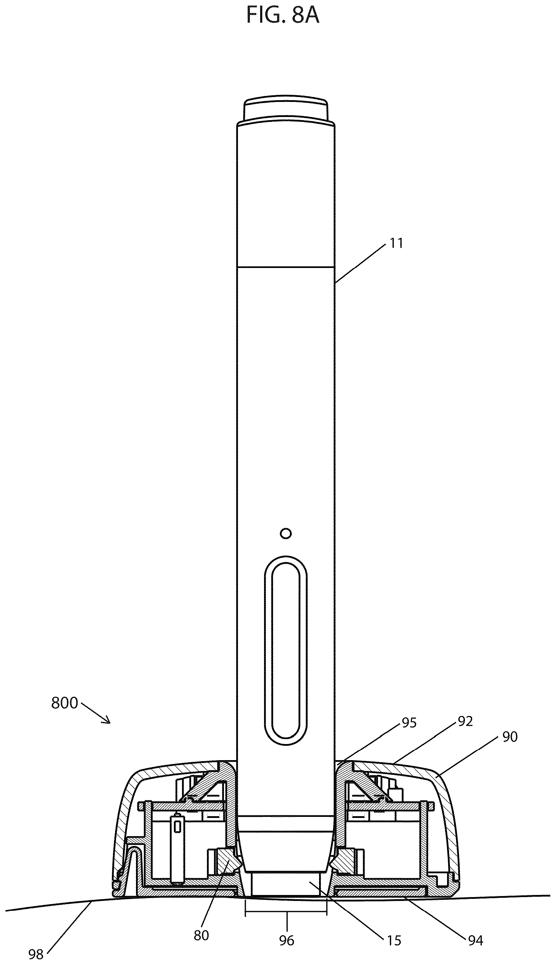

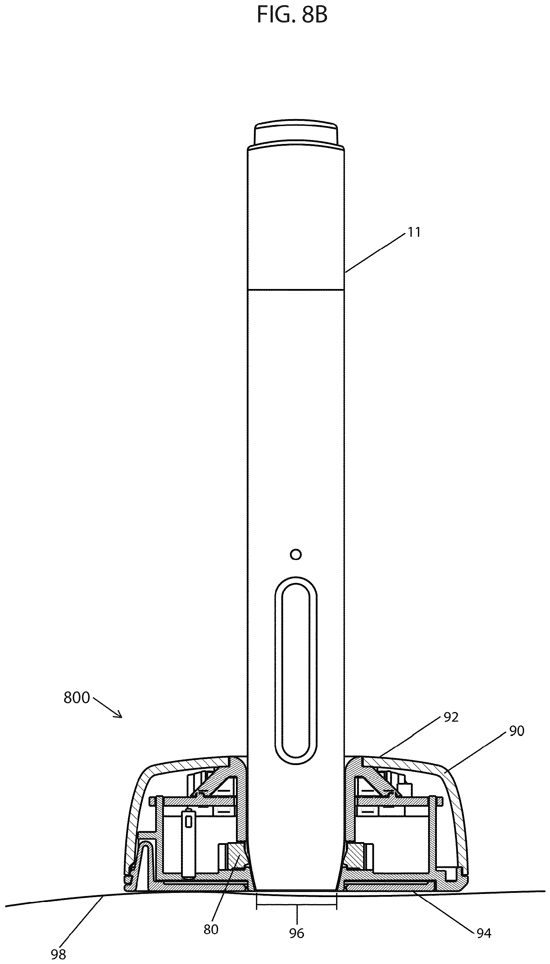

FIG. 8A-8B include a further embodiment 800 of the medicament system, wherein the collateral device 90 includes a top portion 92, a bottom portion 94 for resting against a target area 98 of a user. The collateral device 90 further includes an opening 95 for receiving at least a portion of a medicament device 11. The medicament device 11 may include a safety shield 15 and an injection member (not shown). The collateral device 90 may include a safety shield opening or aperture 96 in the bottom portion 94 configure to receive the distal end of the injection device 11. The sensors 80 are also provided in the embodiment 800 of the system. Sensors 78 as described above may also be provided in this embodiment (not shown). The sensors may detect removal of the cap of the medicament device 11, depression of the needle shield (as shown in FIG. 8B) wherein the contact sensor or contact switch 80 contacts the medicament device 11, and a sensor is provided to indicate contact with the collateral device and the target area of the user. In this embodiment, the medicament device 11 may directly contact the skin of the user via the opening 96, and the injection member may be ejected to deliver medicament to a user. Feedback may be provided to the user about use of the medicament device and/or collateral device of the system via signal output components as described above in regard to the embodiment 700 of FIG. 7.

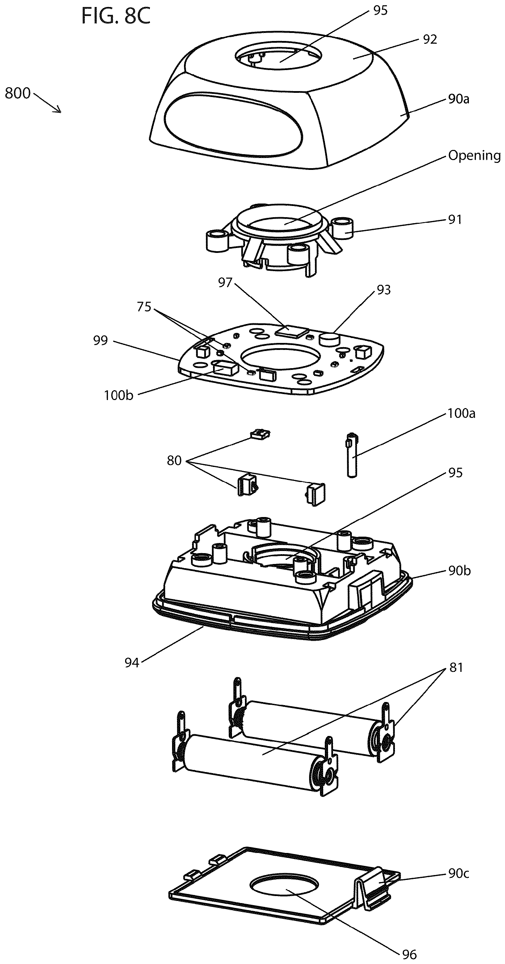

FIG. 8C shows an exploded view of embodiment 800 of the system showing the collateral device upper housing 90a with an opening 95 for the medicament device 11, the upper housing 90a having an upper portion 92, and lower housing 90b having a bottom portion 94 is also provided. A light pipe 91 configured to direct light from the collateral device 90 is shown. A microprocessor 97, a microphone 93, a signal output component 75 (which may include LED, or speaker, or other signal output component) and a blue tooth transmitter 100b are provided, and may be provided on a printed circuit board assembly 99, in a non-limiting embodiment as shown. A storage medium may be further provided in this embodiment. A power source 81 is shown as well as a power source compartment cover 90c having a safety shield aperture 96 for allowing the safety shield to traverse the collateral device 90 to contact the target area of the user during use.

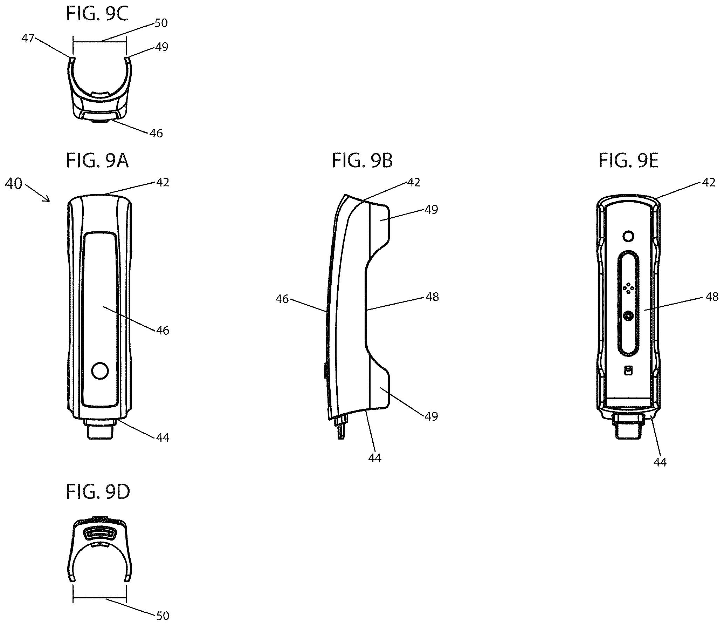

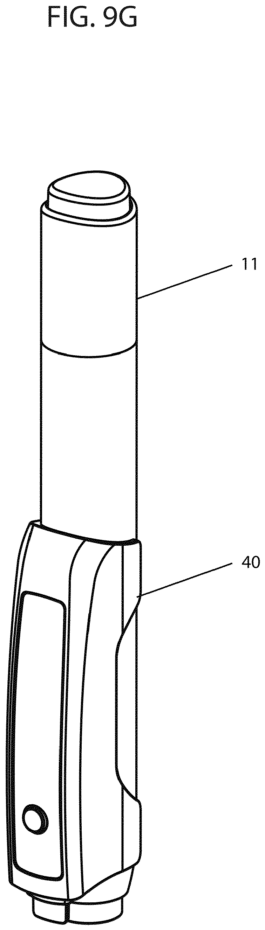

In a further embodiment of the collateral device 40 shown in FIGS. 9A-G, an outer surface 46, an inner surface 48, a top side 42, a bottom side 44, a first end 49 and a second end 49 are provided. A contact element 45 is associated with the bottom side 44 of the collateral device embodiment 40. An attachment component 50 is shown as a combination of the inner surface 48 and the first and second ends 47, 49 of the collateral device 40, in the embodiment shown. The attachment component, as further described herein, may include other forms of attachment, such as an adhesive, a hook and loop fastener, a clip, or as shown in the embodiment of the collateral device 40, a particular shape of the housing corresponding to a shape of the medicament device upon which the collateral device may be attached. The attachment component may include a portion of the housing that is complementary in shape to a portion of the housing of the medicament device as provided in the embodiment of FIGS. 9A-G. FIG. 9G shows attachment of the collateral device 40 onto the medicament device 11. The attachment may be permanent or non-permanent. The collateral device may snap onto the medicament device 11, in a non-limiting embodiment, during use of the medicament device 11, and may be removable and reusable for another medicament device 11.

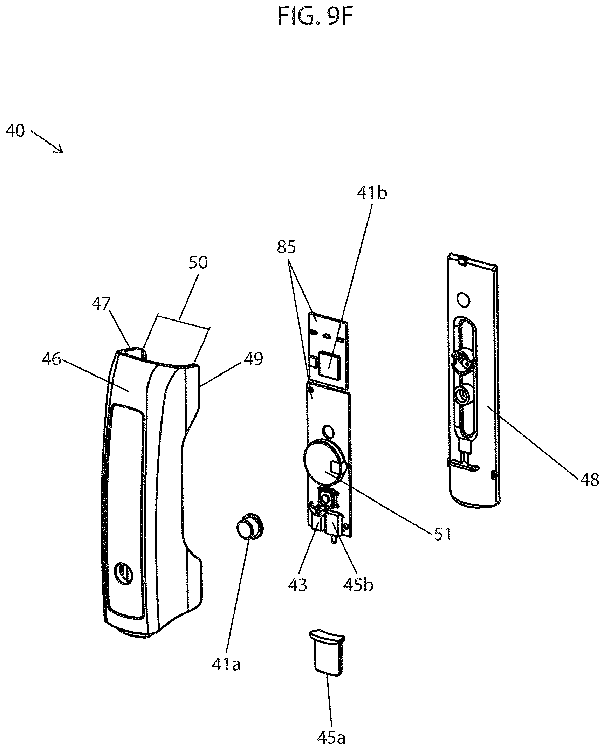

The collateral device 40 includes a microphone 43 and a power source 51 and may further include a blue tooth pairing component 41a, a blue tooth transmitter 41b, and some or all of these components may be associated with a printed circuit board assembly 85 as shown herein. The collateral device 40 further includes one or more signal output components to provide information and/or feedback to a user. The microphone 43 receives sounds from the medicament device 11 during use, and may be used to identify a status of the medicament device 11 as described in other embodiments herein, such as movement of one or more components of the medicament device relative to one another during use of the medicament device. Another example may include a sound that indicates completion of delivery of medicament from the medicament device, which may be picked up by the microphone 43 component, for example. The collateral device embodiment 40 may include a contact element 45a that may interact with a contact switch 45b, in a non-limiting embodiment shown in the exploded view of FIG. 9F. The contact element 45a and contact switch 45b may interact with one another to indicate a contact with the medicament device 11 and the target area of the user and further, to indicate deflection of the safety shield of the medicament device, wherein upon deflection of the safety shield, the contact element 45a is pressed against the target area of the user, and is displaced in a direction toward the contact switch 45b to activate the contact switch 45b. The collateral device 40 may register the status of the medicament device 11. The collateral device 40 may also provide feedback to a user via a signal output component regarding the status of the medicament device 11.

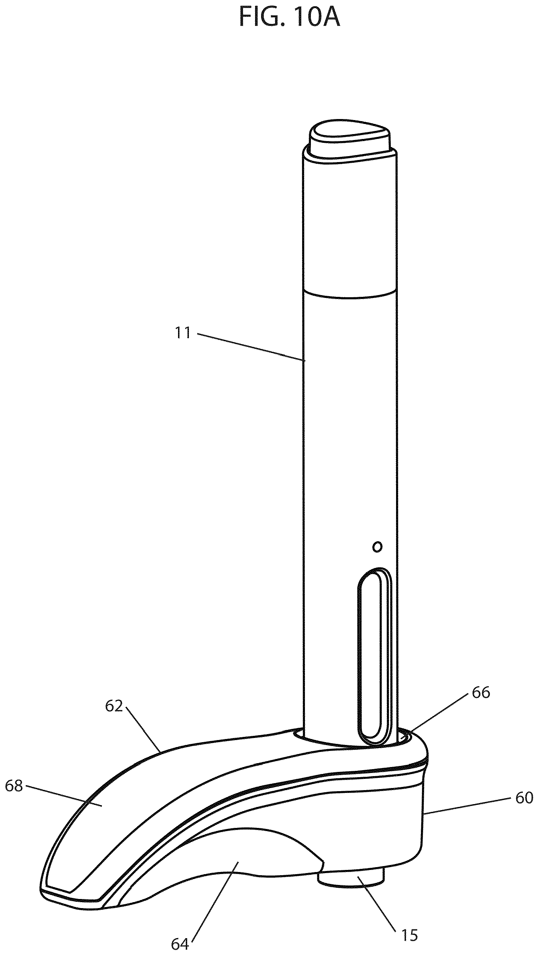

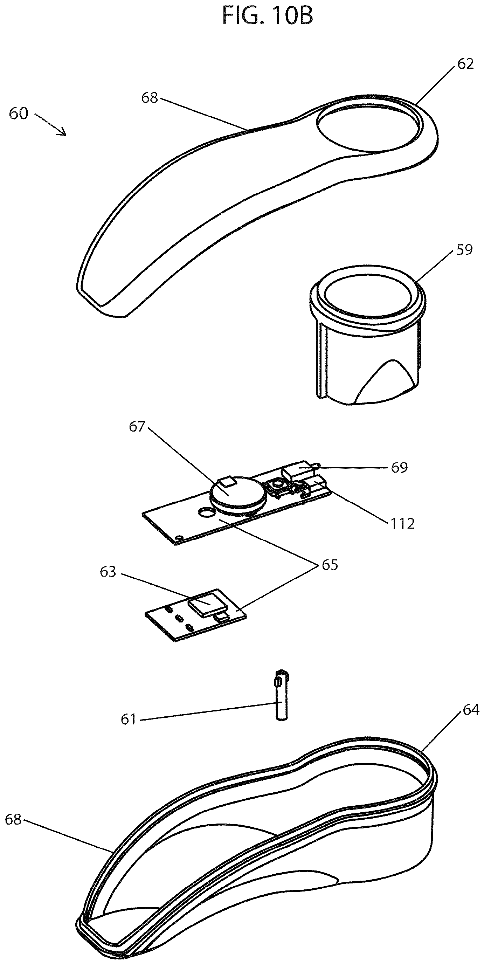

FIG. 10A includes a further embodiment of the collateral device 60 shown in a perspective view with a medicament device 11 being received within an opening 66 of the collateral device 60. FIG. 10B is an exploded view of the collateral device embodiment 60. The embodiment of the collateral device 60 in FIGS. 10A-10B includes a handle portion 68, a top portion 62, and a bottom portion 64. The handle portion 68 may be used to facilitate use of the collateral device 60 and/or the medicament device 11 during an injection, for example. The medicament device 11 is receivable within the opening 66 of the collateral device 60, until the safety shield 15 traverses the collateral device opening 66 to contact the target area of a user as shown in the non-limiting embodiment of FIG. 10A.

The exploded view of FIG. 10B further provides a view of the components of the collateral device embodiment 60, including a light pipe 59, configured to direct light from the collateral device. The light may be provided on a printed circuit board assembly 65 of the collateral device 60 in a non-limiting embodiment. The light may include an LED in a further non-limiting embodiment. The light may provide a feedback to a user of the device during a use of the device 60. The light pipe 59 may direct the light upward through the opening 66 in the top housing 62 of the collateral device 60, in a non-limiting embodiment, such that it can be viewed by a user, for example.

A power source 67 is further provided, and in one non-limiting embodiment, may be provided on the printed circuit board assembly 65. Other components may include a microprocessor, a storage module, other signal output components including a speaker, a display, an olfactory component or a vibratory component, in non-limiting embodiments. One or more sensors 69 may be provided in the collateral device embodiment 60 as described in detail herein. The sensor may include a contact sensor to detect contact with the medicament device when the medicament device is inserted into the collateral device 60. In one embodiment, the sensor 69 may include a contact switch as shown in FIG. 10B, wherein when the medicament device 11 is received within the opening 66, the contact switch is activated to indicate receipt of the medicament device 11 and correct positioning for medicament delivery, in a non-limiting embodiment. In another non-limiting embodiment, the sensor may further include an accelerometer which may identify status of the medicament device 11 as the medicament device is associated with the collateral device 60. The accelerometer may be used to identify a status of the device. The collateral device may further include a blue tooth transmitter 63 and a blue tooth pairing button to pair with and allow transfer of information between the collateral device 60 and other devices. The collateral device 60 also includes a microphone 112 wherein a status of the medicament device 11 may be determined based on the sounds received from the microphone 112 created by movement of the medicament device 11, or movement of components of the medicament device relative to one another during use of the device 11. Information about the status of the medicament device 11 may be provided to the user.

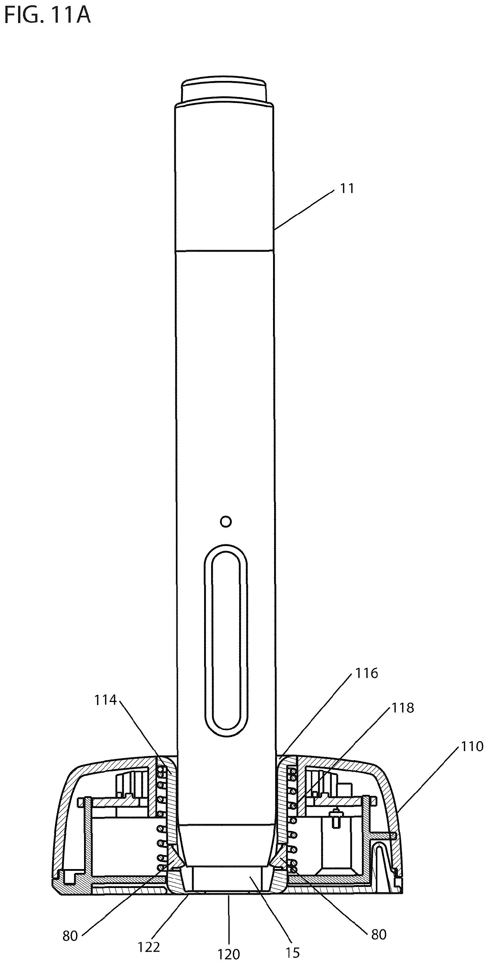

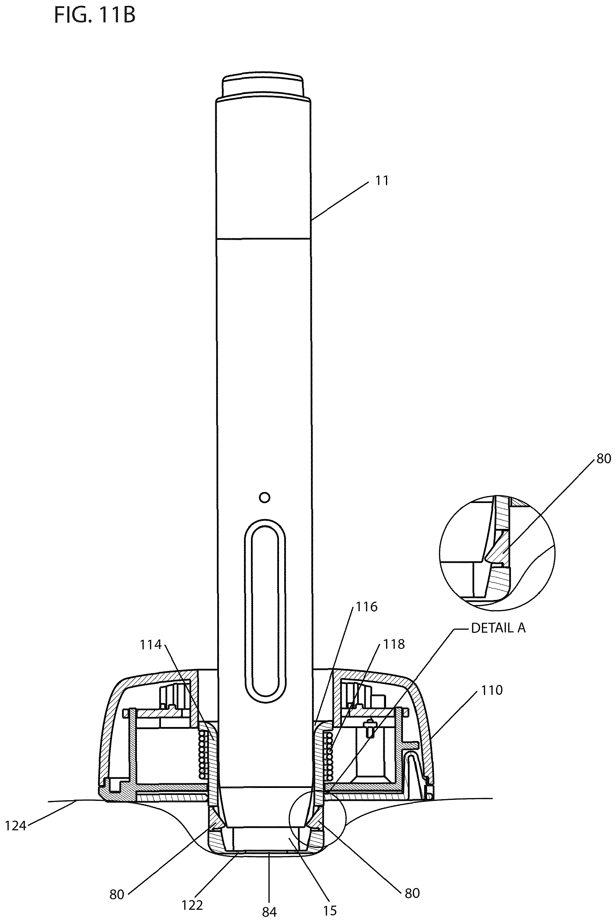

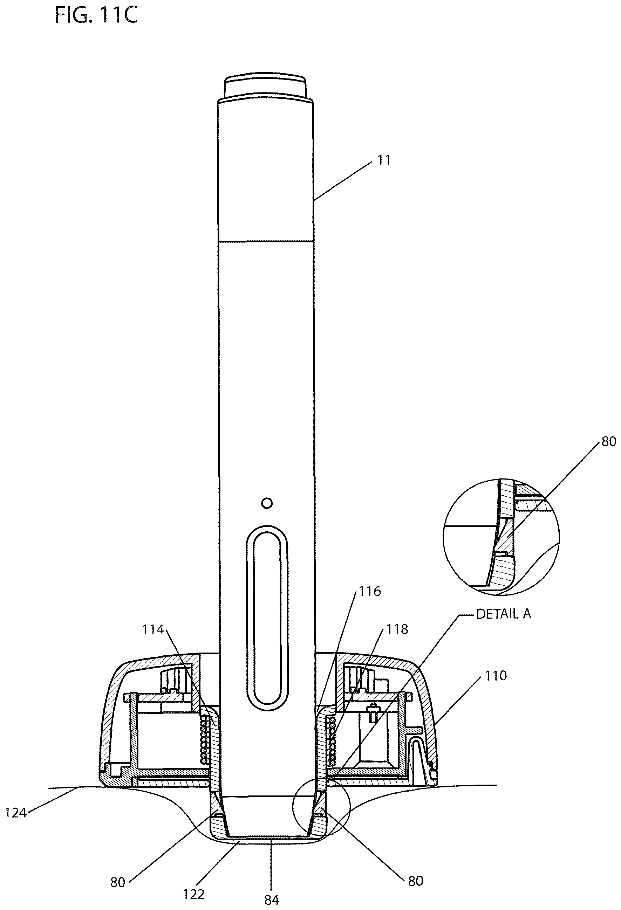

In a further embodiment, in FIG. 11A-C, a collateral device embodiment 110 including an internal element 114 having an opening 116 for receiving a medicament device, a biasing member 118, one or more sensors 80, and an aperture 120 for receiving an injection member of the medicament device 11 is provided. The internal element 114 is slidable relative to the collateral device housing 114, wherein when a bottom portion 122 of the collateral device is against a target area of a user, and a medicament device 11 is inserted into the opening 116 and pressed down toward the target area of the user, the biasing member 118 is deformed and the internal element 114 is displaced relative to the collateral device 110, such that it extends below the collateral device 110. Further movement of the medicament device toward the target area of the user as shown in FIG. 11C allows contact between a sensor 80 and a distal portion of the medicament device 11 when the safety shield 15 of the medicament device is depressed. Actuation of the medicament device 11 may occur following depression of the safety shield. Following delivery of medicament, pressure on the medicament device 11 may be released, and the biasing member 118 may retract the internal element 114 back into the collateral device. Further sensors may be provided between the portion of the collateral device and/or the internal element 114 that contacts the target area of the user to ensure contact there between during use of the device/system. This embodiment allows precise control of the start and stop position of the medicament device 11, and may provide for controlled movement of the medicament device and repeatable use of the sensors, by way of manipulation of the internal element 114. Control over the strength and resistance of the biasing member 118, among the other components of the embodiment may be further provided in this embodiment.

While a number of embodiments of the present invention have been shown and described herein in the present context, such embodiments are provided by way of example only, and not of limitation. Numerous variations, changes and substitutions will occur to those of skill in the art without materially departing from the invention herein. For example, the present invention need not be limited to best mode disclosed herein, since other applications can equally benefit from the teachings of the present invention. Also, in the claims, means-plus-function and step-plus-function clauses are intended to cover the structures and acts, respectively, described herein as performing the recited function and not only structural equivalents or act equivalents, but also equivalent structures or equivalent acts, respectively. Accordingly, all such modifications are intended to be included within the scope of this invention as defined in the following claims, in accordance with relevant law as to their interpretation.

While one or more embodiments of the present invention have been shown and described herein, such embodiments are provided by way of example only. Variations, changes and substitutions may be made without departing from the invention herein. Accordingly, it is intended that the invention be limited only by the scope of the appended claims.

## Claims

1. A medicament system (700, 800) configured to receive and/or communicate information about a medicament device or about a use of the medicament device, or a combination thereof, to a user, said medicament system (700, 800) comprising:
a medicament device (11) comprising a housing ;
a collateral device (40, 60, 70, 90, 110) to provide information to a user, said collateral device (40, 60, 70, 90, 110) comprising an information detecting and/or receiving component;
a signal output component (75);
a microprocessor (73, 97); and
a power source (51, 67, 81);
wherein the collateral device (40, 60, 70, 90, 110) includes a sensor (69, 78, 80, 82)
wherein the signal output component provides an output comprising information about the medicament system and/or a usage of the medicament device and/or medicament system to a user of the system;
**characterized in that**:
the collateral device (40, 60, 70, 90, 110) comprises a collateral device housing comprising an opening (66, 76, 95, 116) for receiving the medicament device;
the medicament device (11) comprises a mechanism comprising one or more mechanical components that move relative to one another to produce a mechanical sound during use;
the information detecting and/or receiving component comprises a microphone (43, 93, 112) configured to receive an audible signal from the medicament device and/or the mechanical sound produced by the mechanism;
the collateral device (40, 60, 70, 90, 110) comprises the signal output component (75), the microprocessor (73, 97) and the power source (51, 67, 81);
the sensor (82) detects contact of the collateral device (40, 60, 70, 90, 110) against the skin of a user;
the collateral device (40, 60, 70, 90, 110) is configured to detect an error condition of the medicament device in response to information received by the microphone or information received by the microphone and information sensed by the sensor, wherein the error condition is incorrect positioning between the device and the user, incorrect contact between the device and the user, an out of order step, and/or a step that exceeds or fails to meet a predetermined time value.

2. The medicament system (700, 800) of claim 1, further comprising an attachment component (38) configured to secure the collateral device to the medicament device.

3. The medicament system (700, 800) of claim 1, wherein the medicament device communicatingly connects to the collateral device via a wired or a wireless connection, wherein said connection provides a communication of power and/or information between the medicament device and the collateral device.

4. The medicament system (700, 800) of claim 1, wherein the medicament device further comprises a removable cap (19).

5. The medicament system (700, 800) of claim 1, wherein the medicament device comprises a safety shield (15).

6. The medicament system (700, 800) of claim 1, wherein the medicament device comprises a shield-activated injection device.

7. The medicament system (700, 800) of claim 1, wherein the medicament device comprises a housing comprising an elongated body and a tapered distal end (13).

8. The medicament system (700, 800) of claim 7, wherein the collateral device housing comprises a top portion (62, 72, 92) and a bottom portion (64, 74, 94), said opening (66, 76, 95) there between.

9. The medicament system (700, 800) of claim 1, wherein the collateral device further comprises a sensor (78) adjacent to the opening, wherein the sensor is configured to detect when the medicament device distal end (13) is received within the opening, wherein contact between the sensor and the distal end of the medicament device confirms that a cap (19) has been removed from the distal end (13) of the medicament device.

10. The medicament system (700, 800) of claim 1, wherein the error condition is a wet injection or an out of sequence operation of the medicament device.

## Patentansprüche

1. Medikamentensystem (700, 800), das dazu ausgebildet ist, Informationen über eine Medikamentenvorrichtung bzw. über eine Verwendung der Medikamentenvorrichtung oder eine Kombination davon zu empfangen und/oder an einen Benutzer zu übermitteln, wobei das Medikamentensystem (700, 800) Folgendes umfasst:
eine Medikamentenvorrichtung (11) mit einem Gehäuse;
eine an der Seite befindliche Vorrichtung (40, 60, 70, 90, 110) zur Bereitstellung von Informationen für einen Benutzer, wobei die an der Seite befindliche Vorrichtung (40, 60, 70, 90, 110) eine Komponente zur Erfassung und/oder zum Empfang von Informationen umfasst;
eine Signalausgabekomponente (75);
einen Mikroprozessor (73, 97); und
eine Stromquelle (51, 67, 81);
wobei die an der Seite befindliche Vorrichtung (40, 60, 70, 90, 110) einen Sensor (69, 78, 80, 82) aufweist,
wobei die Signalausgabekomponente einen Ausgang für einen Benutzer des Systems bereitstellt, der Informationen über das Medikamentensystem und/oder eine Benutzung der Medikamentenvorrichtung und/oder des Medikamentensystems umfasst;
**dadurch gekennzeichnet, dass**:
die an der Seite befindliche Vorrichtung (40, 60, 70, 90, 110) ein Gehäuse der an der Seite befindlichen Vorrichtung umfasst, das eine Öffnung (66, 76, 95, 116) zur Aufnahme der Medikamentenvorrichtung umfasst;
die Medikamentenvorrichtung (11) einen Mechanismus mit einer oder mehreren mechanischen Komponenten umfasst, die sich relativ zueinander bewegen, um während des Gebrauchs ein mechanisches Geräusch zu erzeugen;
die Komponente zur Erfassung und/oder zum Empfang von Informationen ein Mikrophon (43, 93, 112) umfasst, das dazu ausgebildet ist, ein hörbares Signal von der Medikamentenvorrichtung und/oder das von dem Mechanismus erzeugte mechanische Geräusch zu empfangen;
die an der Seite befindliche Vorrichtung (40, 60, 70, 90, 110) die Signalausgabekomponente (75), den Mikroprozessor (73, 97) und die Stromquelle (51, 67, 81) umfasst;
der Sensor (82) den Kontakt der an der Seite befindlichen Vorrichtung (40, 60, 70, 90, 110) gegen die Haut eines Benutzers erfasst;
die an der Seite befindliche Vorrichtung (40, 60, 70, 90, 110) dazu ausgebildet ist, einen Fehlerzustand der Medikamentenvorrichtung in Reaktion auf von dem Mikrophon empfangene Informationen oder von dem Mikrophon empfangene und von dem Sensor erfasste Informationen zu erfassen, wobei es sich bei dem Fehlerzustand um eine falsche Positionierung zwischen der Vorrichtung und dem Benutzer, einen falschen Kontakt zwischen der Vorrichtung und dem Benutzer, einen außerplanmäßigen Schritt und/oder einen Schritt handelt, der einen vorbestimmten Zeitwert überschreitet oder nicht erfüllt.

2. Medikamentensystem (700, 800) nach Anspruch 1, ferner umfassend eine Befestigungskomponente (38), die dazu ausgebildet ist, die an der Seite befindliche Vorrichtung an der Medikamentenvorrichtung zu befestigen.

3. Medikamentensystem (700, 800) nach Anspruch 1, wobei die Medikamentenvorrichtung die an der Seite befindliche Vorrichtung über eine drahtgebundene oder drahtlose Verbindung kommunizierend verbindet, wobei die Verbindung für eine Übermittlung von Energie und/oder Informationen zwischen der Medikamentenvorrichtung und der an der Seite befindlichen Vorrichtung sorgt.

4. Medikamentensystem (700, 800) nach Anspruch 1, wobei die Medikamentenvorrichtung ferner eine abnehmbare Kappe (19) umfasst.

5. Medikamentensystem (700, 800) nach Anspruch 1, wobei die Medikamentenvorrichtung ein Schutzschild (15) umfasst.

6. Medikamentensystem (700, 800) nach Anspruch 1, wobei die Medikamentenvorrichtung eine schildaktivierte Injektionsvorrichtung umfasst.

7. Medikamentensystem (700, 800) nach Anspruch 1, wobei die Medikamentenvorrichtung ein Gehäuse mit einem langgestreckten Körper und einem verjüngten distalen Ende (13) umfasst.

8. Medikamentensystem (700, 800) nach Anspruch 7, wobei das Gehäuse der an der Seite befindlichen Vorrichtung einen oberen Abschnitt (62, 72, 92) und einen unteren Abschnitt (64, 74, 94) umfasst, wobei sich die Öffnung (66, 76, 95) dazwischen befindet.

9. Medikamentensystem (700, 800) nach Anspruch 1, wobei die an der Seite befindliche Vorrichtung ferner einen an die Öffnung angrenzenden Sensor (78) umfasst, wobei der Sensor dazu ausgebildet ist, es zu erfassen, wenn das distale Ende (13) der Medikamentenvorrichtung in der Öffnung aufgenommen ist, wobei ein Kontakt zwischen dem Sensor und dem distalen Ende der Medikamentenvorrichtung bestätigt, dass eine Kappe (19) von dem distalen Ende (13) der Medikamentenvorrichtung entfernt wurde.

10. Medikamentensystem (700, 800) nach Anspruch 1, wobei es sich bei dem Fehlerzustand um eine Nassinjektion oder einen außer der Reihe erfolgenden Betrieb der Medikamentenvorrichtung handelt.

## Revendications

1. Un système pour médicament (700, 800) configuré pour recevoir et/ou communiquer à un utilisateur des informations sur un dispositif pour médicament ou sur une utilisation du dispositif pour médicament, ou une combinaison de ceux-ci, ledit système pour médicament (700, 800) comprenant :
un dispositif pour médicament (11) comprenant un boîtier ;
un dispositif collatéral (40, 60, 70, 90, 110) pour fournir des informations à un utilisateur, ledit dispositif collatéral (40, 60, 70, 90, 110) comprenant un composant de détection et/ou de réception d'informations ;
un composant (75) de délivrance de signal ;
un microprocesseur (73, 97) ; et
une source d'alimentation (51, 67, 81) ;
le dispositif collatéral (40, 60, 70, 90, 110) comprenant un capteur (69, 78, 80, 82),
le composant de délivrance de signal fournissant à un utilisateur du système une sortie qui comprend des informations sur le système pour médicament et/ou sur une utilisation du dispositif pour médicament et/ou du système pour médicament ;
**caractérisé en ce que** :
le dispositif collatéral (40, 60, 70, 90, 110) comprend un boîtier de dispositif collatéral comprenant une ouverture (66, 76, 95, 116) pour recevoir le dispositif pour médicament ;
le dispositif pour médicament (11) comprend un mécanisme comprenant un ou plusieurs composants mécaniques qui se déplacent les uns par rapport aux autres pour produire un son mécanique pendant l'utilisation ;
le composant de détection et/ou de réception d'informations comprend un microphone (43, 93, 112) configuré pour recevoir un signal audible du dispositif pour médicament et/ou le son mécanique produit par le mécanisme ;
le dispositif collatéral (40, 60, 70, 90, 110) comprend le composant (75) de délivrance de signal, le microprocesseur (73, 97) et la source d'alimentation (51, 67, 81) ;
le capteur (82) détecte le contact du dispositif collatéral (40, 60, 70, 90, 110) contre la peau d'un utilisateur ;
le dispositif collatéral (40, 60, 70, 90, 110) est configuré pour détecter une condition d'erreur du dispositif pour médicament en réponse aux informations reçues par le microphone ou aux informations reçues par le microphone et aux informations détectées par le capteur, la condition d'erreur étant un mauvais positionnement entre le dispositif et l'utilisateur, un mauvais contact entre le dispositif et l'utilisateur, une étape mise en œuvre selon un mauvais ordre, et/ou une étape qui dépasse ou ne respecte pas une valeur de temps prédéterminée.

2. Le système pour médicament (700, 800) selon la revendication 1, comprenant en outre un composant de fixation (38) configuré pour fixer le dispositif collatéral au dispositif pour médicament.

3. Le système pour médicament (700, 800) selon la revendication 1, dans lequel le dispositif pour médicament se connecte de manière communicante au dispositif collatéral via une connexion câblée ou sans fil, ladite connexion fournissant une communication d'alimentation et/ou d'informations entre le dispositif pour médicament et le dispositif collatéral.

4. Le système pour médicament (700, 800) selon la revendication 1, dans lequel le dispositif pour médicament comprend en outre un capuchon amovible (19).

5. Le système pour médicament (700, 800) selon la revendication 1, dans lequel le dispositif pour médicament comprend un écran de sécurité (15).

6. Le système pour médicament (700, 800) selon la revendication 1, dans lequel le dispositif pour médicament comprend un dispositif d'injection activé par un bouclier.

7. Le système pour médicament (700, 800) selon la revendication 1, dans lequel le dispositif pour médicament comprend un boîtier comprenant un corps allongé et une extrémité distale conique (13).

8. Le système pour médicament (700, 800) selon la revendication 7, dans lequel le boîtier de dispositif collatéral comprend une partie supérieure (62, 72, 92) et une partie inférieure (64, 74, 94), ladite ouverture (66, 76, 95) étant entre elles.

9. Le système pour médicament (700, 800) selon la revendication 1, dans lequel le dispositif collatéral comprend en outre un capteur (78) adjacent à l'ouverture, le capteur étant configuré pour détecter le moment auquel l'extrémité distale (13) du dispositif pour médicament est reçue à l'intérieur de l'ouverture, un contact entre le capteur et l'extrémité distale du dispositif pour médicament confirmant qu'un capuchon (19) a été retiré de l'extrémité distale (13) du dispositif pour médicament.

10. Le système pour médicament (700, 800) selon la revendication 1, dans lequel la condition d'erreur est une injection humide ou une action sur le dispositif pour médicament faite selon un mauvais ordre.
